(19) Europäisches Patentamt
European Patent Office
Office européen des brevets

(11) **EP 2 527 473 A2**

(12) **EUROPEAN PATENT APPLICATION**

(43) Date of publication:
**28.11.2012 Bulletin 2012/48**

(51) Int Cl.:
*C12Q 1/68* [(2006.01)]     *G01N 33/68* [(2006.01)]

(21) Application number: **12176927.7**

(22) Date of filing: **24.05.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **29.05.2009  US 182545 P**

(62) Document number(s) of the earlier application(s) in accordance with Art. 76 EPC:
**10720116.2 / 2 435 580**

(71) Applicants:
• **The Regents of the University of California**
  **Oakland, CA 94607-5200 (US)**
• **TRUSTEES OF THE UNIVERSITY OF PENNSYLVANIA**
  **Philadelphia, PA 19104 (US)**
• **Emory University**
  **Atlanta, GA 30322 (US)**

(72) Inventors:
• **Seyfert, Vicki**
  **Chevy Chase, MD 20815 (US)**
• **Asare, Adam**
  **Chevy Chase, MD 20815 (US)**
• **Turka, Laurence A**
  **Boston, MD 02118 (US)**
• **Newell, Kenneth**
  **Atlanta, GA 30339 (US)**

(74) Representative: **Campbell, Patrick John Henry**
  **J A Kemp**
  **14 South Square**
  **Gray's Inn**
  **London WC1R 5JJ (GB)**

Remarks:
This application was filed on 18-07-2012 as a divisional application to the application mentioned under INID code 62.

(54) **B cell signature associated with tolerance in transplant recipients**

(57)     The present invention provides methods for predicting the development of tolerance to a transplant, such as a kidney, using molecular markers that have different expression patterns in tolerant transplant recipients, as compared to non-tolerant or healthy, non-recipient controls.

# FIGURE 5

**Description**

CROSS-REFERENCES TO RELATED APPLICATIONS

This application claims priority to US Provisional Application No. 61,182,545, filed May 29, 2009, the disclosure of which is incorporated herein by reference in its entirety.

STATEMENT AS TO RIGHTS TO INVENTIONS MADE UNDER FEDERALLY SPONSORED RESEARCH OR DEVELOPMENT

The invention was made with government support under Grant No. NO1-AI-15416 awarded by the National Institutes of Health. The government has certain rights in this invention.

BACKGROUND OF THE INVENTION

Two decades of advances in immunosuppression have led to vast improvements in both the control of acute rejection and short term graft survival in renal transplantation. However, similar improvements in long-term outcomes have not yet been achieved, and concerns over the morbidity of life-long regimens of immunosuppressive drugs remain (Nankivell et al., N Engl J Med 2003; 349(24):2326-2333; Opelz, Transplantation 1995; 60(11):1220-1224). Establishing long-term allograft acceptance without the requirement for continuous immunosuppression, a condition known as allograft tolerance, is therefore a highly desirable therapeutic goal in kidney transplantation (Lechler et al., Nat Rev Immunol 2003; 3(2):147-158).

Unlike liver transplantation, where it is estimated that up to 20% of recipients may be withdrawn from all immunosuppression (Devlin et al., Hepatology 1998; 27(4):926-933; Mazariegos et al., Transplantation 1997; 63(2):243-249; Ramos et al., 1995; 59(2):212-217; Koshiba et al., Transpl Immunol 2007; 17(2):94-97), tolerance to renal allografts appears to be far less frequent (Koshiba et al., 2007; Brouard et al., Am J Transplant 2005; 5(2):330-340; Roussey-Kesler et al., Am J Transplant 2006; 6(4):736-746; Brouard et al., Proc Natl Acad Sci USA 2007; 104(39):15448-15453). Although tolerance has been achieved in numerous animal models of renal transplantation, attempts to induce long-term allograft tolerance in humans have been far less successful, and may be complicated by the potential loss of the engrafted kidney during immunosuppression minimization or withdrawal (Koshiba et al. 2007; Brouard et al., 2007; Kingsley et al., Transpl Int 2007; 20(10):828-841).

Several recent studies have attempted to identify biomarkers of tolerance in liver and kidney transplantation (Brouard et al., 2007; Seyfert-Margolis V, Turka LA. Marking a path to transplant tolerance. J Clin Invest 2008; 118(8):2684-2686). In liver transplantation, the proportion of γδ T cells (specifically TCR δ1 cells) and the ratio of plasmacytoid to myeloid dendritic cells were shown to be increased in tolerant liver transplant recipients relative to those stable on immunosuppression (Mazariegos et al., Am J Transplant 2003; 3(6):689-696; Martinez-Llordella et al., Am J Transplant 2007; 7(2):309-319). Additionally, specific patterns of expressed genes were shown to be associated with tolerant participants as compared to those stable on immunosuppression and healthy controls (Martinez-Llordella et al., J Clin Invest 2008; 118(8):2845-2857). Similarly, two studies have shown that tolerant kidney transplant recipients have distinct patterns of expressed genes and T-cell receptor gene usage (Brouard et al. 2005; Brouard et al. 2007).

Tolerance biomarkers facilitate prediction of selecting transplant recipients, such as kidney recipients, who are good candidates for withdrawal or minimization of immunosuppressive drugs, thereby minimizing toxicity associated with long-term use of immunosuppressive regimens.

BRIEF SUMMARY OF THE INVENTION

The present invention is based on findings with important implications for the long term management of immunosuppression in kidney allograft recipients. The invention provides a signature of tolerance in transplant recipients, including kidney, liver, and heart recipients. Evidence of increased B cells in a subset of immunosuppressed participants, as well as increases in specific marker genes described herein are useful as an indication, along with stable clinical course, to begin immunosuppression minimization. As immunosuppression therapy is minimized, these same markers can be used to monitor for safety and potentially identify individuals that could be developing a tolerant state. The B cell-centric signature that was uncovered provides new insight regarding the mechanisms of allograft tolerance.

Accordingly, in some embodiments, the invention provides a biomarker array comprising at least one biomarker selected from the group consisting of the biomarkers disclosed in Table X and Table Z. In some embodiments, the array comprises at least 2, 3, 4, 5, 10, 15, 20, or more of the biomarkers disclosed in Table X and Table Z. In some embodiments, the array comprises at least 2, 3, 4, 5, 10, 15, 20, or more of the biomarkers disclosed in Table Z. In some embodiments, the biomarker array comprises IgκV1D-13. In some embodiments, the biomarker array comprises IgκV4-1 and IgκV1D-13. In some embodiments, the biomarker array comprises IgκV4-1, IgλL1, and IgκV1D-13. In some embodiments, the biomarker array does not comprise MS4A1.

In some embodiments, the invention provides methods for predicting tolerance in a transplant recipient for the transplant. In some embodiments, the method comprises: (i) detecting in a biological sample from the transplant recipient the expression level of at least one biomarker selected from the group consisting of the biomarkers disclosed in Table X and Table Z; and (ii) comparing the expression level of the at least one biomarker with the expression level of the same at least one biomarker from a biomarker control, thereby predicting the tolerance of the transplant recipient for the transplant. In some embodiments, the detection step comprises a PCR-based method (e.g., RT-PCR, qPCR). In some embodiments, the detection step comprises use of a biomarker array.

In some embodiments, expression of the biomarker IgκV1D-13 is detected and compared. In some embodiments, expression of the biomarkers IgκV4-1 and IgκV1D-13 is detected and compared. In some embodiments, expression of the biomarkers IgκV4-1, IgλLI, and IgκV1D-13 is detected and compared. In some embodiments, the biomarker control is an individual or group of non-tolerant transplant recipients and expression level of the at least one biomarker is increased at least two-fold over the control.

In some embodiments, the method comprises adjusting the course of immunosuppressive therapy for the transplant recipient depending on whether the transplant recipient is predicted to be tolerant of the transplant. For example, if the transplant recipient is predicted to be tolerant, immunosuppressive therapy can be reduced (minimized) or even stopped. If however, the transplant recipient demonstrates a non-tolerant biomarker signature, immunosuppressive therapy can be restored to or continued at a standard level. If the transplant recipient has not yet begun immunosuppressive therapy, therapy can be avoided if the transplant recipient is predicted to be tolerant. If the transplant recipient has yet to receive the transplant, the recipient can be started with a minimal course of immunosuppressive therapy or no immunosuppressive therapy at all post-transplant. In some embodiments, the method is repeated periodically after transplantation to monitor the transplant recipient and further adjust the course of immunosuppressive therapy if necessary.

In some embodiments, the invention provides methods for determining a course of immunosuppressive therapy for a transplant recipient. In some embodiments, the method comprises: (i) detecting in a biological sample from the transplant recipient the expression level of at least one biomarker selected from the group consisting of the biomarkers disclosed in Table X and Table Z; (ii) comparing the expression level of the at least one biomarker with the expression levels of the same at least one biomarker from a biomarker control; (iii) predicting whether the transplant recipient will be tolerant of the transplant based on the comparison in step (ii); and (iv) adjusting the course of immunosuppressive therapy for the transplant recipient depending on whether the transplant recipient is predicted to be tolerant of the transplant.

In some embodiments, expression of the biomarker IgκV1D-13 is detected and compared. In some embodiments, expression of the biomarkers IgκV4-1 and IgκV1D-13 is detected and compared. In some embodiments, expression of the biomarkers IgκV4-1, IgλL1, and IgκV1D-13 is detected and compared. In some embodiments, the biomarker control is an individual or group of non-tolerant transplant recipients and expression level of the at least one biomarker is increased at least two-fold over the control.

In some embodiments, the method is carried out once immunosuppressive therapy has been initiated. In some embodiments, the method is carried out more than once, *e.g.,* to monitor the transplant recipient over time, and, if applicable, in different immunosuppressive therapy regimes. In some embodiments, immunosuppressive therapy is reduced if the transplant recipient is predicted to be tolerant of the transplant. In some embodiments, no immunosuppressive therapy is prescribed, *e.g.,* immunosuppressive therapy is ceased, if the transplant recipient is predicted to be tolerant of the transplant. If the transplant recipient demonstrates a non-tolerant biomarker signature, immunosuppressive therapy can be restored to or continued at a standard level.

In some embodiments, the invention provides methods for predicting tolerance in a transplant recipient, said method comprising: (i) detecting in a biological sample from the transplant recipient the expression level of at least one biomarker selected from the group consisting of: CD20, FOXP3, Perforin, and CD3; and (ii) comparing the expression level of the at least one biomarker with the expression level of the same at least one biomarker from a biomarker control; thereby predicting the tolerance of the transplant recipient. In some embodiments, the biological sample comprises urine sedimentary cells. In some embodiments, the detecting step comprises a PCR-based method *(e.g.,* RT-PCR, qPCR). In some embodiments, the detecting step comprises using an array. In some embodiments, the detecting step comprises a protein detection method *(e.g.,* flow cytometry or other immunoassay). Any one, two, three, or four of the biomarkers can be detected alone or in any combination, *e.g.,* CD20 and FOXP3, or FOXP3 and Perforin, or CD20, CD3, and FOXP3, etc.

In some embodiments, this urine sedimentary cell method can be used alone or in combination with the other predictive methods described herein to determine a course of immunosuppressive therapy for a transplant recipient. For example, the at least one biomarker selected from CD20, FOXP3, Perforin, and CD3 can be detected before transplant and after, and/or at various times post-transplant. If the transplant recipient is predicted to be tolerant, immunosuppressive therapy can be minimized or ceased. If however, the transplant recipient demonstrates a non-tolerant biomarker signature, immunosuppressive therapy can be restored to a higher or standard level.

In some embodiments, the invention provides methods for predicting tolerance of a transplant recipient for the transplant, the method comprising, (i) detecting in a biological sample from the transplant recipient the amount of at least one B cell population in the sample; and (ii) comparing the amount of said at least one B cell population to the amount of the same at least one B cell population in a biological sample from a B cell control, wherein the at least one B cell population is selected from: total B cells, naïve B cells, memory B cells, transitional B cells, and CD86+ B cells. In some embodiments, the detecting step comprises fluorescence activated cell sorting (FACS). In some embodiments, the biological sample is a whole blood sample, or a blood sample where the red blood cells have been removed. Any one, two, three, four, or five of the B cell populations can be detected alone or in any combination. For example, naive and transitional B cells, or total B cells, naive and transitional B cells, or total B cells, transitional, and memory B cells, etc.

In some embodiments, B cells are detected based on expression of CD19. In some embodiments, naïve B cells are detected based on expression of CD 19, IgM, and IgD, and low or undetectable expression of CD27. In some embodiments, memory B cells are detected based on expression of CD19, IgM, IgD, and CD27. In some embodiments, CD86+ B cells are detected based on expression of CD19 and CD86. In some embodiments, transitional B cells are detected based on expression of CD19, CD38, CD24, and IgD. In some embodiments, transitional B cells are further tested for expression of IL-10, TGF-$\beta$, and/ or other immunomodulatory cytokines. In some embodiments, smaller subpopulations of B cells are detected and compared (e.g., T1 and T2 transitional B cells ($CD38^+CD24^+$), switched memory ($CD27^+IgD^-$), $CD27^-$ memory ($CD27^-$ $IgD^-$), naive and T3 transitional ($CD27^-IgD^+$), unswitched memory ($CD27^+IgD^+$), and plasmablasts ($CD27^+CD38^+IgD^-$)).

In some embodiments, this B cell detection method can be used alone or in combination with the other predictive methods described herein to determine a course of immunosuppressive therapy for a transplant recipient, and/ or to monitor the patient post-transplant. For example, the at least one B cell population can be detected before transplant and after, and/ or at various times post-transplant. If the transplant recipient is predicted to be tolerant, immunosuppressive therapy can be minimized or ceased. If however, the transplant recipient demonstrates a non-tolerant biomarker signature, immunosuppressive therapy can be restored to a higher or standard level.

In some embodiments, the predictive methods described herein can be used alone or in any combination to predict whether a transplant recipient will be tolerant of transplanted cells, e.g., using minimized or no immunosuppressive therapy. In some embodiments, the prediction can be made based on detection and comparison of at least one biomarker from Tables X and Z, as well as on detection and comparison of the amount of at least one B cell population. In some embodiments, the prediction can be made based on the detection and comparison of at least one biomarker selected from Tables X and Z and the detection and comparison of at least one biomarker selected from CD20, FOXP3, CD3, and perforin. In some embodiments, the prediction can be made based on detection and comparison of the amount of at least one B cell population, in combination with detection and comparison of at least one biomarker selected from CD20, FOXP3, CD3, and perforin. In some embodiments, all three methods are carried out.

In some embodiments, the predictions can be used alone or in any combination to determine a course of immunosuppressive therapy and/ or monitor the transplant recipient over time. In some embodiments, more than one predictive method is used more than once. In some embodiments, more than one predictive method is used once, *e.g.,* to determine an initial course of immunosuppressive therapy, but fewer of the methods, *e.g.,* only one method is used to monitor the transplant recipient and determine if immunosuppressive therapy should be adjusted.

For the methods described herein, the biomarker or B cell control can be selected from the group consisting of: (i) an individual non-tolerant transplant recipient; (ii) a group of non-tolerant transplant recipients; (iii) an individual healthy non-transplant recipient; (iv) a group of healthy non-transplant recipients; and (v) a sample from the transplant recipient taken at a different time. The controls for different tests can be obtained from different sources *(e.g.,* healthy non-transplant recipients for one biomarker control and non-tolerant transplant recipients for another biomarker control), or from the same source *(e.g.,* non-tolerant transplant recipients for both the biomarker and B cell controls). More than one control can be used for any of the methods described herein.

The above described methods can be practiced with the aid of computers and automated systems. In some embodiments, the detection step *(e.g.,* detecting biomarker expression levels, levels of cell surface markers, etc.) is carried out automatically, aided by a computer. In some embodiments, a computer records the data detected in the previous step. In some embodiments, the comparing step is carried out by a computer. In some embodiments, a computer assigns a prediction or diagnosis to the sample from the transplant recipient, *e.g.,* tolerant, non-tolerant, responding to immunosuppressive therapy, etc. In some embodiments, the computer comprises data for appropriate controls, *e.g.,* non-tolerant transplant recipients or healthy non-recipients. In this way, the data stored on the computer can act as the control, alleviating the need to obtain control samples or maintain control sample stocks to be used with each new transplant recipient sample.

BRIEF DESCRIPTION OF THE DRAWINGS

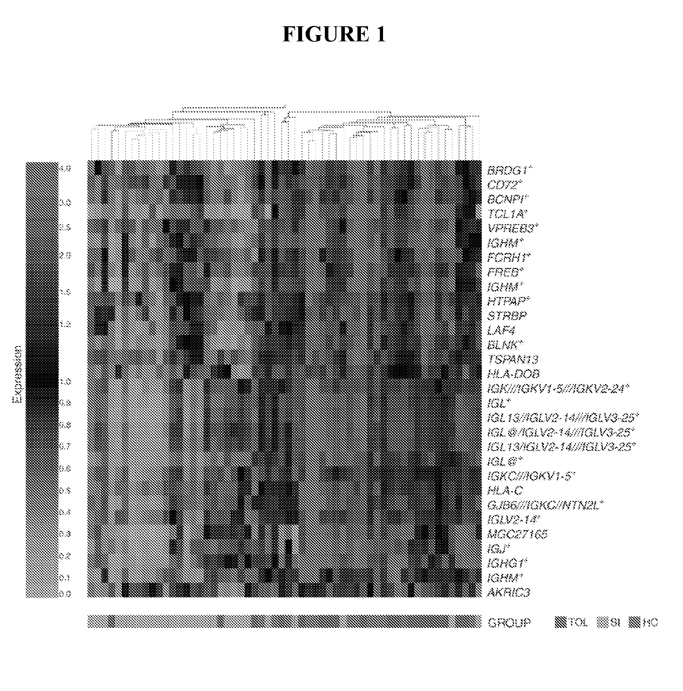

**Figure 1:** TOL participants exhibit unique expression patterns compared with SI participants. Hierarchical clustering of the 30 genes differentially expressed between TOL versus SI (fold change >2.0 overexpressed in the TOL group). *TUBB2A* not shown. B cell-specific genes are indicated by "+".

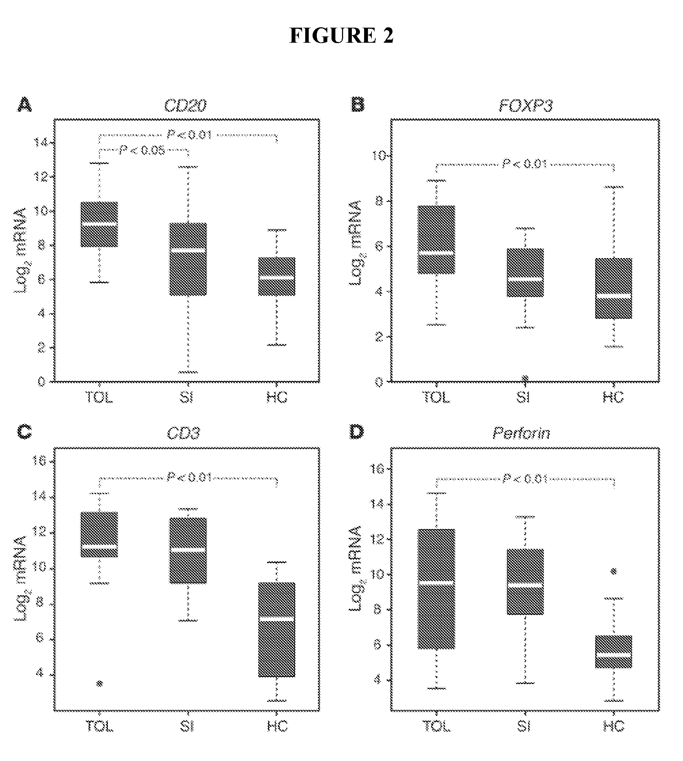

**Figure 2:** Real-time PCR gene expression analyses of urine sedimentary cells. (A) Higher CD20 expression in TOL than in SI and HC participants. (B) Increased FOXP3 expression in TOL than in HC participants. (C) Higher CD3 expression in TOL than in HC participants. (D) Higher Perforin expression in TOL than in HC participants. Boxes depict IQR; whiskers denote 1.5 x IQR; values beyond this range are considered outliers and shown as circles. *P* values are shown for statistically significant differences.

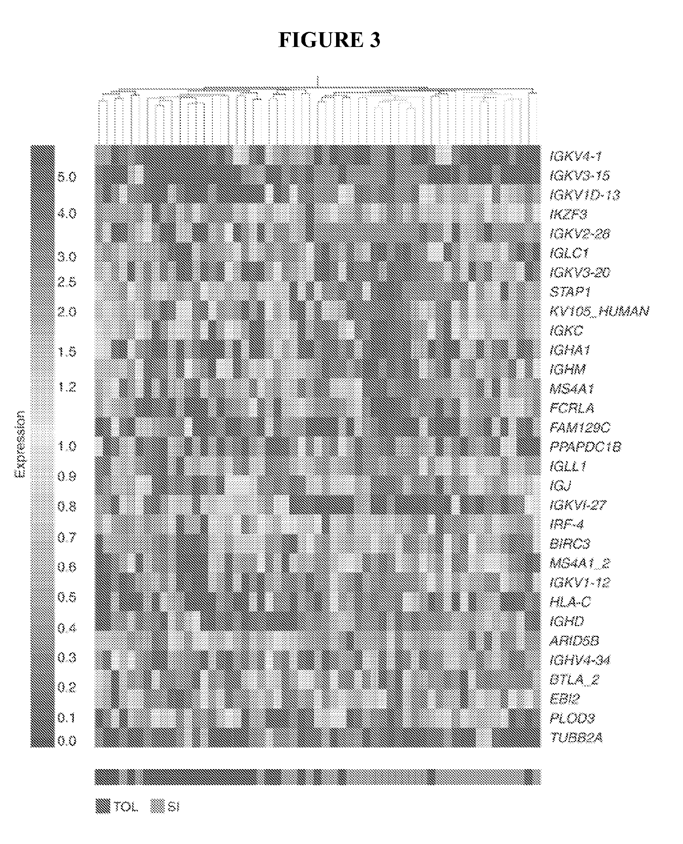

**Figure 3:** Multiplex RT-PCR of peripheral blood identified 31 genes with significantly different numbers of mRNA copies between TOL-TRN and SI-TRN groups. The transcript relative expression levels are shown on the heat map.

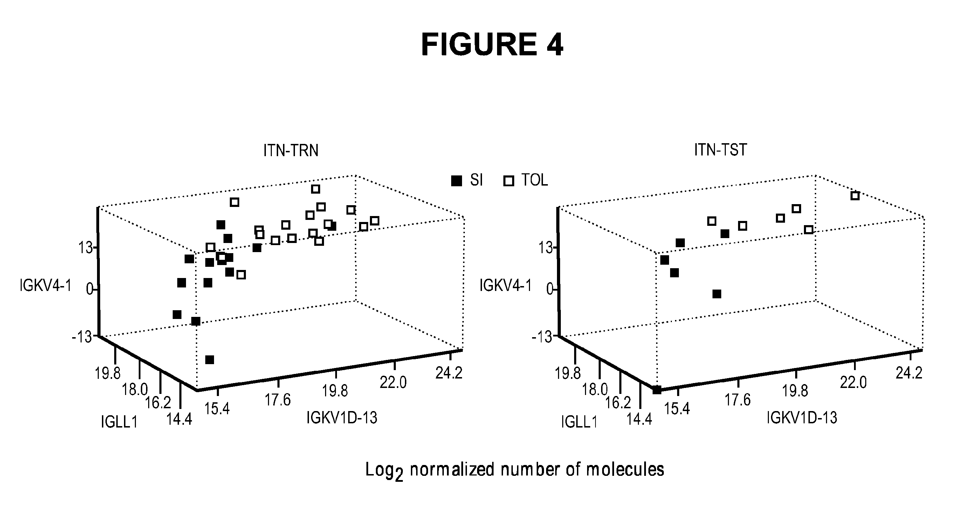

**Figure 4.** Transcripts that best distinguish TOL from SI. Multiplex RT-PCR gene expression levels of IGKV1D-13, IGKV4-1, and IGLL1 for the 19 TOL-TRN and 24 SI-TRN samples, and for the 6 TOL-TST and 6 SI-TST samples, in $\log_2$ normalized number of molecules.

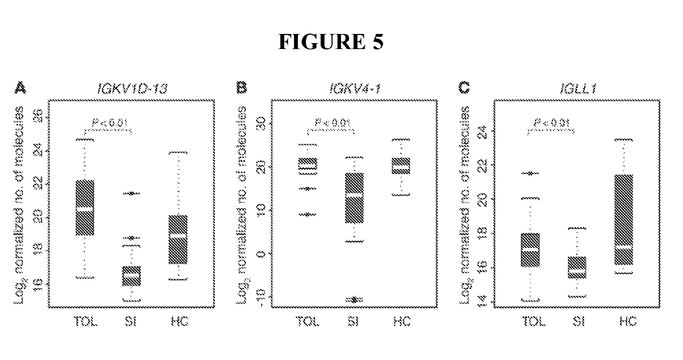

**Figure 5:** Box plots of log2 normalized mRNA copy numbers for the three genes found to be the best classifiers among the 31 identified as differentially expressed. (A) IGKV1D-13. (B) IGKV4-1. (C) IGLL1. Genes were derived from LDA, where they were found to have the best predictive value for TOL (Tables 6 and 7). Boxes depict IQR; whiskers denote 1.5 x IQR; values beyond this range are shown as outliers. *P* values are shown for statistically significant differences.

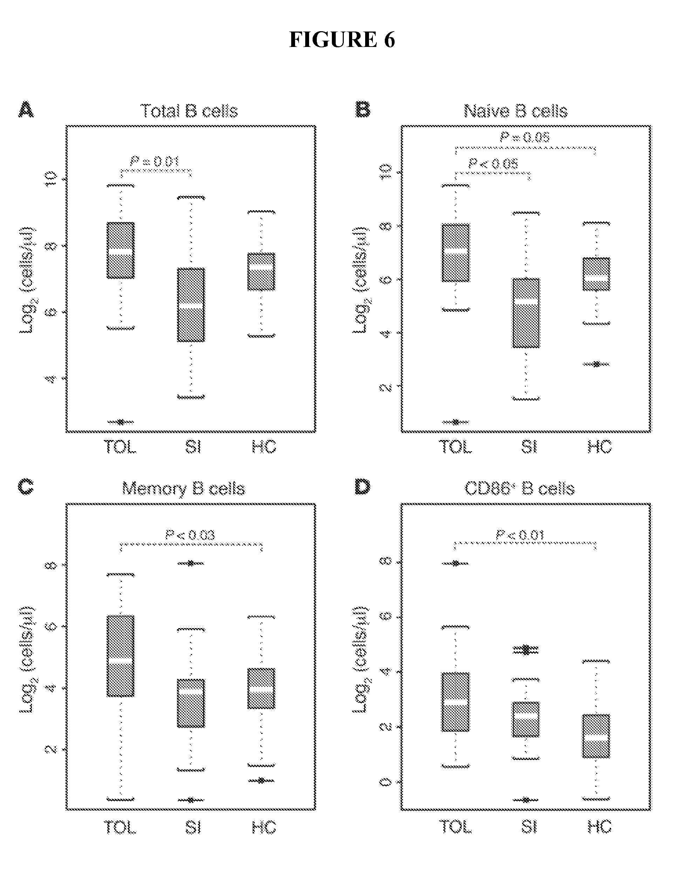

**Figure 6:** Five-color flow cytometry of whole blood samples shows different numbers of B cell subsets. (A) Total B cells, as defined by $CD19^+$ cells. The total B cell counts for TOL, SI, and HC cohorts were 287, 120, and 176 cells/$\mu$l, respectively. (B) Naive B cells, as defined by $CD9^+CD27^-IgM^+IgD^+$ cells. The naive B cell counts for TOL, SI, and HC cohorts were 190, 61, and 90 cells/$\mu$l, respectively. (C) Memory B cells, as defined by $CD27^+IgM^+IgD^+$. The mean numbers for the memory B cell subset were 54.2 and 20.8 cells/$\mu$l for TOL and HC, respectively. (D) $CD86^+$ B cells, as defined by $CD86^+CD19^+$. Mean numbers of $CD86^+CD19^+$ B cells for TOL and HC cohorts were 22 and 4.5 cells/$\mu$l, respectively. All values are shown as $\log_2$ absolute numbers, a calculation of the percent of lymphocyte gate multiplied by the total wbc count obtained from the same sample on a Coulter Counter. Boxes depict IQR; whiskers denote 1.5 x IQR; values beyond this range are shown as outliers. *P* values are shown for statistically significant differences.

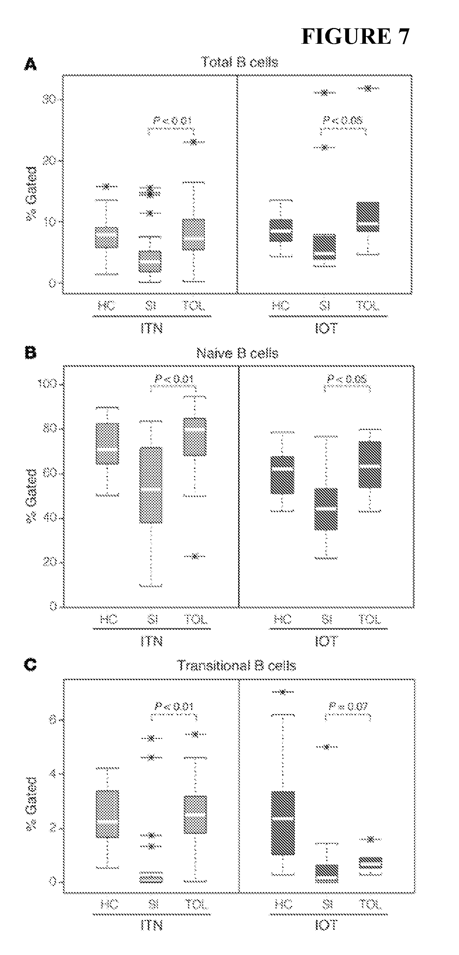

**Figure 7:** 9-color flow cytometry of frozen PBMCs from both ITN and European IOT samples. (A) Total B cells, expressed as the percent $CD19^+$ cells in the lymphocyte gate. (B) Naive B cells, defined as $CD19^+CD27^- IgD^+$, shown as the percent of the total $CD19^+$ gate. (C) Transitional B cells, defined as $CD19^+CD38^+CD24^+IgD^+$, shown as the percent of the total $CD19^+$ gate. Boxes depict IQR; whiskers denote 1.5 x IQR; values beyond this range are shown as outliers. *P* values are shown for statistically significant differences.

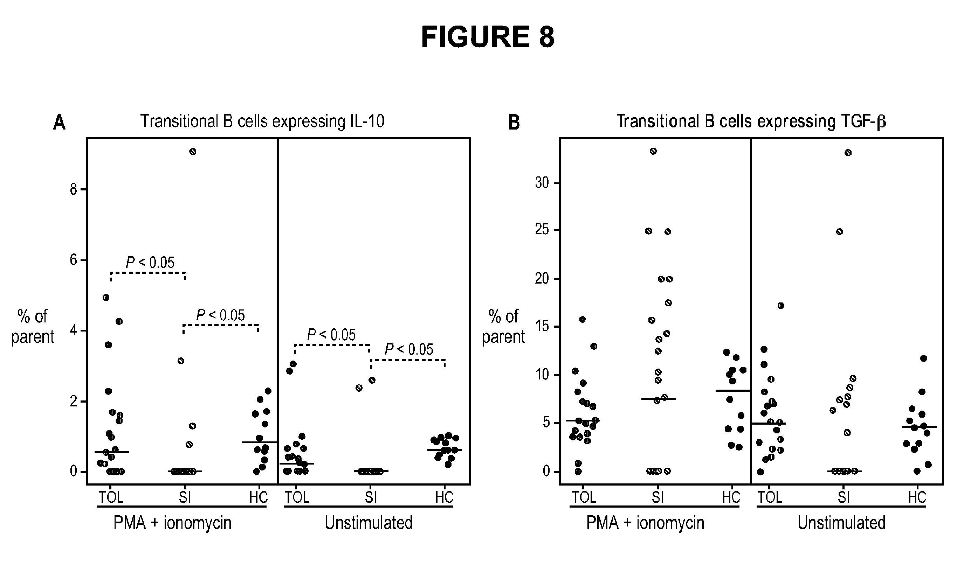

**Figure 8:** Intracellular cytokine staining of sorted B cells. (A) Intracellular cytokine flow cytometric measures of IL-10 in unstimulated transitional B cells and in B cells stimulated with PMA and ionomycin revealed little to no transitional B cells expressing IL-10 in SI participants, with greater numbers of IL-10-expressing transitional B cells in TOL and HC participants in both unstimulated and PMA and ionomycin-stimulated groups. (B) Intracellular flow cytometric cytokine measures of TGFβ in unstimulated transitional B cells and transitional B cells stimulated with PMA and ionomycin revealed no differences in the number of TGFβ-expressing cells in the unstimulated or PMA and ionomycin-stimulated groups. Horizontal lines indicate median.

DETAILED DESCRIPTION OF THE INVENTION

**I. Introduction**

Establishing long-term allograft acceptance without the requirement for continuous immunosuppression, a condition known as allograft tolerance, is a highly desirable therapeutic goal in solid organ transplantation. Determining which recipients would benefit from withdrawal or minimization of immunosuppression would be greatly facilitated by biomarkers predictive of tolerance.

The inventors recruited the largest known cohort (N=25) of tolerant kidney transplant recipients, twenty of whom ceased taking immunosuppression due to medication non-adherence. Selected immune parameters were compared in (1) tolerant kidney transplant recipients (TOL), (2) kidney transplant recipients with stable allograft function while on immunosuppression (SI), and (3) healthy (non-transplanted) controls (HC).

The tolerant cohort was well matched with donors for HLA molecules, and exhibited increased numbers of total B cells and expression of B cell differentiation and activation genes as compared with subjects receiving immunosuppression. This B cell signature was associated with upregulation of CD20 mRNA in urine sediment cells and elevated numbers of peripheral blood naive and transitional B cells in tolerant participants compared with those receiving immunosuppression. The differentially expressed B-cell genes highly predictive of tolerance could be narrowed down to a set of two or three which included IGKV1D-13. The B cell-related biomarkers are shown to be highly predictive of tolerance in a new test set of patients. These markers are strong candidates for clinical testing as a means to predict which kidney transplant recipients will benefit from minimization or withdrawal of immunosuppression, and for monitoring status during immunosuppression withdrawal.

The invention also provides tolerance (TOL) biomarkers that are predictive of an individual's tolerance for a transplant. The biomarkers disclosed in Tables X and Z are expressed at a higher level in TOL kidney transplant recipients compared to SI kidney transplant recipients, e.g., expression is at least 1.5, 2, 3, 5, 7, or 10-fold higher in TOL recipients than in SI recipients. Biomarkers also include markers that are expressed at a lower level in TOL kidney transplant recipients compared to SI kidney transplant recipients, for instance, at least 1.5, 2, 3, 5, 7, or 10-fold lower expression. Further, a marker can be a molecule that is differentially modified or synthesized in TOL kidney transplant recipients or in SI kidney transplant recipients, for instance, a molecule that contains deletions, additions or mutations in comparison to the molecule expressed on a normal cell.

## II. Definitions

The following abbreviations are used throughout:

TOL: tolerant kidney transplant recipient
SI: kidney transplant recipient receiving standard immunosuppression therapy
CAN: chronic allograft nephropathy
HC: healthy, non-recipient control
TRN: training
TST: test
HLA: human leukocyte antigen
Cr: serum creatine
FACS: fluorescence activated cell sorting
ITN: Immune Tolerance Network
IOT: Indices of Tolerance

The term "marker" or "biomarker" refers to a molecule that is expressed in a cell, expressed on the surface of a cell or secreted by a cell and which is useful for predicting or diagnosing a particular condition. A biomarker is typically a protein, nucleic acid, carbohydrate, or lipid. Markers include phosphorylated, methylated, glycosylated, and otherwise modified forms of these molecules. For the purposes of the present invention, biomarkers are useful for predicting tolerance of an individual for a kidney transplant, for diagnosis of tolerance of an individual for a kidney transplant, for providing a prognosis of tolerance of an individual for a kidney transplant, and for preferential targeting of a pharmacological agent(s) in the induction of tolerance of an individual for a kidney transplant.

It will be understood by the skilled artisan that each marker can be used singly or in combination. The presently disclosed markers can also be used with other markers from other gene expression signatures for any of the uses, *e.g.,* prediction, diagnosis, or prognosis of immunotolerance, disclosed herein.

The term "tolerant" refers to an individual with a reduced or absent immune response to a specific antigen or group of antigens. In the context of the invention, an individual is considered tolerant if he or she does not reject *(i.e.,* mount a significant immune response against) transplanted cells. In some cases, the tolerant individual does not reject transplanted cells, even in the absence of immunosuppressive therapy. In the context of the invention, an individual is considered "non-tolerant" if the individual rejects transplanted cells. Non-tolerant individuals include those where rejection is controlled using immunosuppressive therapy *(e.g.,* standard immunosuppression), as well as those that are experiencing an active immune response against transplanted cells.

Typically, the level of a polynucleotide or polypeptide of interest will be detected in a "biological sample." A "biological sample" refers to a cell or population of cells or a quantity of tissue or fluid from an animal. Most often, the sample has been removed from an animal, *e.g.,* a human. "Biological sample" includes sections of tissues such as biopsy and autopsy samples, and frozen sections taken for histologic purposes. Biological samples also include blood and blood fractions or products *(e.g.,* serum, plasma, platelets, red blood cells, and the like), sputum, lymph tissue, cultured cells, *e.g.,* primary cultures, explants, and transformed cells, or the fractions from stool, urine, etc. A biological sample is typically obtained from a eukaryotic organism, most preferably a mammal such as a primate *e.g.,* chimpanzee or human; cow; dog; cat; a rodent, *e.g.,* guinea pig, rat, mouse; rabbit; bird; reptile; or fish. Typically, a "biological sample" will contain cells from the animal, but the term can also refer to noncellular biological material, such as noncellular fractions of blood, saliva, or urine, that can be used to measure tolerance-associated polynucleotide or polypeptide levels.

The term "rejection" refers to a state in which a transplanted organ or tissue is not accepted by the body of the recipient. Rejection results from the recipient's immune system attacking the transplanted organ or tissue. Rejection can occur days to weeks after transplantation (acute) or months to years after transplantation (chronic). Acute rejection can occur to some degree in all transplants (except those between identical twins). Tissues such as the kidney or the liver which are highly vascularized (rich in blood vessels), are often the earliest victims of acute rejection. Episodes of acute rejection occur in around 60-75% of first kidney transplants. Chronic rejection refers to cases of transplant rejection where the rejection is due to a poorly understood chronic inflammatory and immune response against the transplanted tissue.

"Immunosuppressive therapy" refers to an act that reduces the activation or efficacy of the immune system. In transplant biology it is most often associated with the use of chemotherapeutics or biological agents which suppress immune function. An exemplary list of such agents includes: calcineurin inhibitors *(e.g.,* cyclosporin, tacrolimus), mammalian target of rapamycin (mTOR) inhibitors *(e.g.,* sirolimus, everolimus), anti-proliferative agents *(e.g.,* azathioprine, mycophenolic acid), corticosteroids *(e.g.,* prednisone, prednisolone, hydrocortisone) and antibodies *(e.g.,* basiliximab, daclizumab). Combined use of a corticosteroid, calcineurin inhibitor and an anti-proliferative agent is referred to as triple therapy.

A "minimized" or "minimal" course of immunosuppressive therapy is one that provides the lowest dose of immunosuppressive therapeutics possible to avoid an immune response. For example, a course of therapy will be considered minimized if the dose or number of immunosuppressive drugs given to a patient is lower than would normally be given to a like patient with similar health profile. As an example, a minimized therapy regime can refer to treatment with only one immunosuppressive drug, such as rapamycin or prednisone, as opposed to a standard multi-drug regime. The minimized therapy can also refer to a reduced dose of the selected immunosuppressive drug. The dose of immunosupressive drugs can be progressively reduced over time in combination with careful patient observation for signs of rejection. Standard monitoring tools are described herein, and include creatinine clearance and glomerular filtration rate.

A "biopsy" refers to the process of removing a tissue sample for diagnostic or prognostic evaluation, and to the tissue specimen itself. Any biopsy technique known in the art can be applied to the diagnostic and prognostic methods of the present invention. The biopsy technique applied will depend on the tissue type to be evaluated *(e.g.*, skin, colon, prostate, kidney, bladder, lymph node, liver, bone marrow, blood cell, *etc.),* among other factors. Representative biopsy techniques include, but are not limited to, excisional biopsy, incisional biopsy, needle biopsy, surgical biopsy, and bone marrow biopsy. A diagnosis or prognosis made by endoscopy or fluoroscopy can require a "core-needle biopsy", or a "fine-needle aspiration biopsy" which generally obtains a suspension of cells from within a target tissue. Biopsy techniques are discussed, for example, in Harrison's Principles of Internal Medicine, Kasper, et al., eds., 16th ed., 2005, Chapter 70, and throughout Part V.

The terms "overexpress," "overexpression" or "overexpressed" or "upregulated" interchangeably refer to a protein or nucleic acid (RNA) that is transcribed or translated at a detectably greater level as compared to a control. The term includes overexpression due to transcription, post transcriptional processing, translation, post-translational processing, cellular localization *(e.g.,* organelle, cytoplasm, nucleus, cell surface), and RNA and protein stability, as compared to a control. Overexpression can be detected using conventional techniques for detecting mRNA *(i.e.,* RT-PCR, PCR, hybridization) or proteins *(i.e.,* ELISA, immunohistochemical techniques). Overexpression can be 10%, 20%, 30%, 40%, 50%, 60%, 70%, 80%, 90% or more in comparison to a normal cell. In certain instances, overexpression is 1-fold, 2-fold, 3-fold, 4-fold or more higher levels of transcription or translation in comparison to a control.

The terms "underexpress," "underexpression" or "underexpressed" or "downregulated" interchangeably refer to a protein or nucleic acid that is transcribed or translated at a detectably lower level as compared to a control. The term includes underexpression due to transcription, post transcriptional processing, translation, post-translational processing, cellular localization *(e.g.,* organelle, cytoplasm, nucleus, cell surface), and RNA and protein stability, as compared to a control. Underexpression can be detected using conventional techniques for detecting mRNA *(i.e.,* RT-PCR, PCR, hybridization) or proteins *(i.e.,* ELISA, immunohistochemical techniques). Underexpression can be 10%, 20%, 30%, 40%, 50%, 60%, 70%, 80%, 90% or less in comparison to a control. In certain instances, underexpression is 1-fold, 2-fold, 3-fold, 4-fold or more lower levels of transcription or translation in comparison to a control.

The term "differentially expressed" or "differentially regulated" refers generally to a protein or nucleic acid that is overexpressed (upregulated) or underexpressed (downregulated) in one sample compared to at least one other sample, in the context of the present invention.

A "control" sample or value refers to a sample that serves as a reference, usually a known reference, for comparison to a test sample. For example, a test sample can be taken from a patient of unknown tolerant status and compared to control samples from a known tolerant patient, a known SI patient, and a known normal (non-recipient) individual. A control can also represent an average value gathered from a population of similar individuals, e.g., tolerant patients with a similar medical background, or non-recipients of the same age, weight, etc. A control value can also be obtained from the same individual, *e.g.,* from an earlier-obtained sample, prior to disease, or prior to transplant. In some cases, the control can be compared within an individual or between individuals by comparing expression of the disclosed biomarkers with that of house-keeping genes, which remain relatively stable in the tissue sample tested *(e.g.,* peripheral blood, IgM+/ IgD+ B cells, etc.).

One of skill in the art will understand which controls are valuable in a given situation and be able to analyze data based on comparisons to control values. Controls are also valuable for determining the significance of data. For example, if values for a given parameter are widely variant in controls, variation in test samples will not be considered as significant.

A "recipient" is an individual that has received or will receive transplanted cells or tissue as part of a medically-supervised therapeutic plan. A "donor" is an individual that provides the cells or tissue. A "non-recipient" is an individual that has not received transplanted material.

A "transplant," as used herein, refers to cells, tissue, or an organ that is introduced into an individual. The source of the transplanted material can be cultured cells, cells from another individual, or cells from the same individual *(e.g.,* after the cells are cultured *in vitro).* Exemplary organ transplants are kidney, liver, heart, lung, and pancreas.

Populations of B cells are described herein. These populations are commonly defined by expression of certain cell surface markers, which can be detected using flow cytometry *(e.g.,* FACS), as well as more traditional gene expression detection methods *(e.g.,* RT-PCR or immunoassay). B cells can be identified by expression of CD 19 (CD19+). Naïve B cells are identified as CD19+/ CD27-/ IgM+/ IgD+. Transitional B cells are identified as CD19+/CD38+/ CD24+/ IgD+. Memory B cells are identified as CD19+/ CD27+/ IgM+/ IgD+.

The amount of a given B cell population can be expressed in a number of ways, as will be appreciated by one of skill in the art. For example, the amount of a population can be expressed as a comparative percentage *(e.g.,* of total B cells or total white blood cells), an absolute number, or a concentration *(e.g.,* cells/ ml). B cell samples are generally obtained from whole blood or cellular blood fractions, though B cells can be obtained from transplanted tissue, lymph, lymph nodes or other immune organs, urine, etc.

The terms "identical" or percent "identity," in the context of two or more nucleic acids or polypeptide sequences, refer to two or more sequences or subsequences that are the same or have a specified percentage of amino acid residues or nucleotides that are the same *(i. e.,* about 60% identity, preferably 65%, 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters described below, or by manual alignment and visual inspection *(see, e.g.,* NCBI web site at ncbi.nlm.nih.gov/BLAST/ or the like). Such sequences are then said to be "substantially identical." This definition also refers to, or may be applied to, the compliment of a test sequence. The definition also includes sequences that have deletions and/or additions, as well as those that have substitutions. As described below, the preferred algorithms can account for gaps and the like. Preferably, identity exists over a region that is at least about 25 amino acids or nucleotides in length, or more preferably over a region that is 50-100 amino acids or nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

A "comparison window," as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well-known in the art. Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection *(see, e.g.,* Current Protocols in Molecular Biology (Ausubel et al., eds. 1987-2005, Wiley Interscience)).

A preferred example of algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990), respectively. BLAST and BLAST 2.0 are used, with the parameters described herein, to determine percent sequence identity for the nucleic acids and proteins of the invention. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra).* These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectation (E) of 10, and the BLOSUM62 scoring matrix *(see* Henikoff & Henikoff, Proc. Natl. Acad. Sci. USA 89:10915 (1989)) alignments (B) of 50, expectation (E) of 10, M=5, N=-4, and a comparison of both strands.

"Nucleic acid" refers to deoxyribonucleotides or ribonucleotides and polymers thereof in either single- or double-stranded form, and complements thereof. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs).

Unless otherwise indicated, a particular nucleic acid sequence also implicitly encompasses conservatively modified variants thereof (e.g., degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); Rossolini et al., Mol. Cell. Probes 8:91-98 (1994)). The term nucleic acid is used interchangeably with gene, cDNA, mRNA, oligonucleotide, and polynucleotide.

A particular nucleic acid sequence also implicitly encompasses "splice variants" and nucleic acid sequences encoding truncated forms of a protein. Similarly, a particular protein encoded by a nucleic acid implicitly encompasses any protein encoded by a splice variant or truncated form of that nucleic acid. "Splice variants," as the name suggests, are products of alternative splicing of a gene. After transcription, an initial nucleic acid transcript may be spliced such that different (alternate) nucleic acid splice products encode different polypeptides. Mechanisms for the production of splice variants vary, but include alternate splicing of exons. Alternate polypeptides derived from the same nucleic acid by read-through transcription are also encompassed by this definition. Any products of a splicing reaction, including recombinant forms of the splice products, are included in this definition. Nucleic acids can be truncated at the 5' end or at the 3' end. Polypeptides can be truncated at the N-terminal end or the C-terminal end. Truncated versions of nucleic acid or polypeptide sequences can be naturally occurring or recombinantly created.

A "label" or a "detectable moiety" is a composition detectable by spectroscopic, photochemical, biochemical, immunochemical, chemical, or other physical means. For example, useful labels include $^{32}$P, fluorescent dyes, electron-dense reagents, enzymes (e.g., as commonly used in an ELISA), biotin, digoxigenin, or haptens and proteins which can be made detectable, e.g., by incorporating a radiolabel into the peptide or used to detect antibodies specifically reactive with the peptide.

The nucleic acids of the differentially expressed genes of this invention or their encoded polypeptides refer to all forms of nucleic acids (e.g., gene, pre-mRNA, mRNA) or proteins, their polymorphic variants, alleles, mutants, and interspecies homologs that (as applicable to nucleic acid or protein): (1) have a sequence that has greater than about 60% identity, 65%, 70%, 75%, 80%, 85%, 90%, preferably 91%, 92%, 93%, 94%, 95%, 96%, 97%, 98% or 99% or greater identity, over a region of at least about 25, 50, 100, 200, 500, 1000, or more nucleotides or amino acids, to a sequence described herein; (2) specifically bind to antibodies, *e.g.,* polyclonal antibodies, raised against an immunogen comprising a referenced amino acid sequence, immunogenic fragments thereof, and conservatively modified variants thereof; (3) specifically hybridize under stringent hybridization conditions to a nucleic acid; (4) have a nucleic acid sequence that has greater than about 95%, preferably greater than about 96%, 97%, 98%, 99%, or higher nucleotide sequence identity, preferably over a region of at least about 25, 50, 100, 200, 500, 1000, or more nucleotides, to a reference nucleic acid sequence. A polynucleotide or polypeptide sequence is typically from a mammal including, but not limited to, primate, e.g., human; rodent, *e.g.,* rat, mouse, hamster; cow, pig, horse, sheep, or any mammal. The nucleic acids and proteins of the invention include both naturally occurring or recombinant molecules. Truncated and alternatively spliced forms of these antigens are included in the definition.

### III. Therapies for transplant patients

The therapeutic management of the transplant patient with immunosuppressants occurs in two phases: the first phase, often referred to as the "induction phase" and the second phase termed the "maintenance phase." The induction phase may last as long as four months following transplant surgery. Maintenance immuno-suppression is the key to prevention of acute and chronic rejections throughout the life of the graft.

Two types of induction strategies are often used to avoid early acute rejection: antibody-based therapy and aggressive early immunosuppression. Antibody-based therapy uses monoclonal *(e.g.,* muromonab-CD3) or polyclonal antibodies or anti-CD25 antibodies *(e.g.,* basiliximab, daclizumab) and is administered in the early post-transplant period (up to 8 wk). Antibody-based therapy allows for avoidance or dose reduction of calcineurin inhibitors, possibly reducing the risk of nephrotoxicity. These agents are effective for preventing acute rejections, although the anti-CD25 antibodies may require concurrent administration of calcineurin inhibitors. The adverse effect profile of the polyclonal and monoclonal antibodies limits their use in some patients. Patients at highest risk of rejection may receive rabbit antithymocyte globulin (Thymoglobulin).

Aggressive early immunosuppression uses maintenance drugs at higher doses to achieve the strongest immunosuppressive effect soon after transplantation. Approximately 50% of patients do not receive antibody therapy at the time of transplantation. The highest doses of calcineurin inhibitors place patients at increased risk of nephrotoxicity and may not be the best strategy for patients at the highest risk for rejection.

For patients with stable graft function, individual components of the treatment regimen can be gradually diminished or completely discontinued. In most patients, some degree of immunosuppression is continued throughout the life of the transplanted organ. Episodes of acute cellular rejection have occurred after the cessation of medication even 20 years after transplantation. The present invention provides a means to detect those individuals that can be safely removed from immunosuppressive treatment regimens.

### IV. Monitoring kidney transplant patients for rejection

A drastic rise in creatinine may indicate acute rejection, especially in the first year following transplant. A gradual rise over a longer period of time is a sign of chronic rejection (e.g., chronic allograft nephropathy (CAN)). Chronic rejection is also associated with proteinuria. Generally, any high level of creatinine that persists or increases warrants a biopsy. The rise could be a sign of rejection or of the primary kidney disease.

The diagnosis of acute rejection relies on clinical data, including patient signs and symptoms, laboratory testing and ultimately a tissue biopsy. Generally the pathologist looks for three main histological features. First, the presence of T-cells infiltrating the transplanted tissue; these may be accompanied by a heterogeneous collection of other cell types including eosinophils, plasma cells and neutrophils. Determining the proportions of these cell types may be helpful in diagnosing the exact type of rejection. Secondly, evidence of structural injury to the transplanted tissue; the characteristics of this injury will depend on the type of tissue being transplanted. Lastly, injury to the blood vessels in the transplanted tissue.

In some transplant programs, kidney transplant recipients with normal creatinine levels are biopsied. Rejection can occur without a raise in creatinine levels. If abnormalities are noted in the first biopsy, subsequent biopsies are preformed at 3-month intervals. These abnormalities can frequently be controlled with appropriate changes in immunosuppressive medication.

### V. Predictive, diagnostic, and prognostic methods

The present invention provides methods of predicting, diagnosing or providing prognosis of tolerance by detecting the expression of markers over-expressed in a transplant recipient tolerant of the transplant in comparison to a transplant recipient on immunosuppression, or healthy control or a transplant recipient with chronic allograft rejection/ damage. Prediction and diagnosis involve determining the level of one or more tolerance biomarker polynucleotide or the corresponding polypeptides in a patient or patient sample and then comparing the level to a baseline or range. Typically, the baseline value is representative of levels of the polynucleotide or nucleic acid in a transplant recipient on immuno-suppression and/or healthy person with no known history of disease/dysfunction in that organ, and/or kidney transplant recipient with chronic allograft nephropathy, as measured using a biological sample. Variation of levels of a polynucleotide or corresponding polypeptides of the invention from the baseline range (either up or down) indicates that the patient is likely to be tolerant of the transplanted tissue.

As used herein, the term "prediction" refers to providing a relative assessment of the chance that a patient is or may be tolerant of the transplanted kidney or tissue. As used herein, the term "providing a prognosis" refers to providing a prediction of the probable likelihood of tolerance. The methods can also be used to devise a suitable therapy for inducing or maintaining tolerance to the transplanted kidney or discontinuing all or some of the immuno-suppressant therapies.

As used herein, the term "diagnosis" refers to detecting the propensity for development of tolerance to a donated kidney(s). Any method of diagnosis has some risk of returning false positives and false negatives. Any one method of diagnosis typically does not provide 100% accuracy.

Analysis of a nucleic acid marker can be performed using techniques known in the art including, without limitation, microarrays, polymerase chain reaction (PCR)-based analysis, sequence analysis, and electrophoretic analysis. A non-limiting example of a PCR-based analysis includes a Taqman® available from Applied Biosystems. Non-limiting examples of electrophoretic analysis include slab gel electrophoresis such as agarose or polyacrylamide gel electrophoresis, capillary electrophoresis, and denaturing gradient gel electrophoresis.

Microarray methods are generally described in Hardiman, "Microarrays Methods and Applications: Nuts & Bolts," DNA Press, 2003; and Baldi et al., "DNA Microarrays and Gene Expression: From Experiments to Data Analysis and Modeling," Cambridge University Press, 2002.

In one set of embodiments, the hybridizations are performed on a solid support. For example, probes that selectively hybridize to specific biomarker transcripts can be spotted onto a surface. Conveniently, the spots are placed in an ordered pattern, or array, and the placement of the probes on the array is recorded to facilitate later correlation of results. The nucleic acid samples are then hybridized to the array. In one configuration, the multiplicity of nucleic acids (or other moieties) is attached to a single contiguous surface or to a multiplicity of surfaces juxtaposed to each other.

In an array format a large number of different hybridization reactions can be run essentially "in parallel." This provides rapid, essentially simultaneous, evaluation of a number of hybridizations in a single test run. Methods of performing hybridization reactions in array based formats are well known to those of skill in the art *(see, e.g.,* U.S. Patent No. 6,040,138; Pastinen (1997) Genome Res. 7: 606-614; Jackson (1996) Nature Biotechnology 14:1685; Chee (1995) Science 274: 610; WO 96/17958).

The simple spotting approach has been automated to produce high density spotted arrays *(see, e.g.,* U.S. Patent No: 5,807,522). This patent describes the use of an automated systems that taps a microcapillary against a surface to deposit a small volume of a biological sample. The process is repeated to generate high density arrays. Arrays can also be produced using oligonucleotide synthesis technology. Thus, for example, U.S. Patent No. 5,143,854 and PCT patent publication Nos. WO 90/15070 and 92/10092 teach the use of light-directed combinatorial synthesis of high density oligonucleotide arrays.

Many methods for immobilizing nucleic acids on a variety of solid surfaces are known in the art. A wide variety of organic and inorganic polymers, as well as other materials, both natural and synthetic, can be employed as the material for the solid surface. Illustrative solid surfaces include, *e.g.,* nitrocellulose, nylon, glass, quartz, diazotized membranes (paper or nylon), silicones, polyformaldehyde, cellulose, and cellulose acetate. In addition, plastics such as polyethylene, polypropylene, polystyrene, and the like can be used. Other materials which may be employed include paper, ceramics, metals, metalloids, semiconductive materials, cermets or the like. In addition, substances that form gels can be used. Such materials include, e.g., proteins (*e.g.*, gelatins), lipopolysaccharides, silicates, agarose and polyacrylamides. Where the solid surface is porous, various pore sizes may be employed depending upon the nature of the system.

Target elements of various sizes, ranging from 1 mm diameter down to 1 $\mu$m can be used. Smaller target elements containing low amounts of concentrated, fixed probe DNA are used for high complexity comparative hybridizations since the total amount of sample available for binding to each target element will be limited. Thus it is advantageous to have small array target elements that contain a small amount of concentrated probe DNA so that the signal that is obtained is highly localized and bright. Such small array target elements are typically used in arrays with densities greater than $10^4/cm^2$. Relatively simple approaches capable of quantitative fluorescent imaging of 1 $cm^2$ areas have been described that permit acquisition of data from a large number of target elements in a single image *(see, e.g.,* Wittrup, Cytometry 16: 206-213, 1994).

Arrays on solid surface substrates with much lower fluorescence than membranes, such as glass, quartz, or small beads, can achieve much better sensitivity. Substrates such as glass or fused silica are advantageous in that they provide a very low fluorescence substrate, and a highly efficient hybridization environment. Covalent attachment of the target nucleic acids to glass or synthetic fused silica can be accomplished according to a number of known techniques, using commercially available reagents. For instance, materials for preparation of silanized glass with a number of functional groups are commercially available or can be prepared using standard techniques (see, *e.g.,* Gait (1984) Oligonucleotide Synthesis: A Practical Approach, IRL Press, Wash., D.C.). Quartz cover slips, which have at least 10-fold lower autofluorescence than glass, can also be silanized.

Alternatively, the samples can be placed in separate wells or chambers and hybridized in their respective well or chambers. The art has developed robotic equipment permitting the automated delivery of reagents to separate reaction chambers, including "chip" and microfluidic techniques, which allow the amount of the reagents used per reaction to be sharply reduced. Chip and microfluidic techniques are taught in, for example, U. S. Patent No. 5,800,690, Orchid, "Running on Parallel Lines" New Scientist, Oct 25, 1997, McCormick, et al., Anal. Chem. 69:2626-30 (1997), and Turgeon, "The Lab of the Future on CD-ROM?" Medical Laboratory Management Report. Dec. 1997, p.1. Automated hybridizations on chips or in a microfluidic environment are contemplated methods of practicing the invention.

Analysis of the biomarkers of the invention can also be achieved using routine techniques such as reverse-transcriptase polymerase chain reaction (RT-PCR), or any other methods based on hybridization to a nucleic acid sequence that is complementary to a portion of the marker coding sequence (e.g., slot blot hybridization) are also within the scope of the present invention. General nucleic acid hybridization methods are described in Anderson, "Nucleic Acid Hybridization," BIOS Scientific Publishers, 1999.

Methods of quantitative amplification are well known to those of skill in the art. Detailed protocols for quantitative PCR are provided, *e.g.,* in Innis et al. (1990) PCR Protocols, A Guide to Methods and Applications, Academic Press, Inc. N.Y.). The known nucleic acid sequences for the genes listed herein is sufficient to enable one of skill to routinely select primers to amplify any portion of the gene.

The hybridized nucleic acids are typically detected by detecting one or more labels attached to the sample or probe nucleic acids. The labels may be incorporated by any of a number of means well known to those of skill in the art. Means of attaching labels to nucleic acids include, for example nick translation or end-labeling *(e.g.* with a labeled RNA) by phosphorylating *(e.g.,* with a kinase) of the nucleic acid and subsequent attachment (ligation) of a nucleic acid linker joining the sample nucleic acid to a label *(e.g.,* a fluorophore). A wide variety of linkers for the attachment of labels to nucleic acids are also known. In addition, intercalating dyes and fluorescent nucleotides can also be used.

Detectable labels suitable for use in the present invention include any composition detectable by spectroscopic, photochemical, biochemical, immunochemical, electrical, optical or chemical means.

A fluorescent label is useful because it provides a very strong signal with low background. It is also optically detectable at high resolution and sensitivity through a quick scanning procedure. The nucleic acid samples can all be labeled with a single label, *e.g.,* a single fluorescent label. Alternatively, different nucleic acid samples can be simultaneously hybridized where each nucleic acid sample has a different label. For instance, one target could have a green fluorescent label and a second target could have a red fluorescent label. The scanning step will distinguish cites of binding of the red label from those binding the green fluorescent label. Each nucleic acid sample (target nucleic acid) can be analyzed independently from one another.

Gas chromatography or mass spectroscopy can be used to detect the marker by analyzing either nucleic acid or protein. Any antibody-based technique for determining a level of expression of a protein of interest can be used to detect a biomarker. For example, immunoassays such as ELISA, Western blotting, flow cytometry, immunofluorescence, and immunohistochemistry can be used to detect a protein in patient samples.

Protein expression of the disclosed tolerance biomarkers can be analyzed according to the methods of the invention. Polypeptides encoded by the genes described herein can be detected and/or quantified by any methods known to those of skill in the art. Samples can be from any biological source, including *e.g.,* tissue biopsies and blood, etc. Antibodies to the gene products listed herein can be obtained commercially or using standard methods.

Such methods can also be used to detect cell surface proteins, *e.g.,* to determine cell type. Cell type can be important so that gene expression *(e.g.,* biomarker expression) is only determined in an appropriate subset of cells. Cell type can be determined using known methods, *e.g.,* morphology, size, immunoassay, flow cytometry (FACS), ELISA, fluorescence microscopy, or by nucleic acid based methods. In the methods of the invention, detection of immune cells involved in transplant rejection can be important. To this end, B cell, T cell, and other immune cell populations can be determined using markers known in the art, and described herein. For example, B cells can be detected by detecting CD19 on the cell surface. Naive B cells also express IgD, but not CD27. Transitional B cells express CD38, CD24, and IgD. Memory B cells express CD27, IgM, and IgD.

Immunoassays also often use a labeling agent to specifically bind to and label the complex formed by the antibody and antigen. The labeling agent may itself be one of the moieties comprising the antibody/antigen complex. Thus, the labeling agent may be a labeled polypeptide or a labeled antibody that binds the protein of interest. Alternatively, the labeling agent may be a third moiety, such as a secondary antibody, that specifically binds to the antibody/ antigen complex (a secondary antibody is typically specific to antibodies of the species from which the first antibody is derived). Other proteins capable of specifically binding immunoglobulin constant regions, such as protein A or protein G may also be used as the labeling agent. These proteins exhibit a strong non-immunogenic reactivity with immunoglobulin constant regions from a variety of species *(see, e.g.,* Kronval et al., J. Immunol. 111:1401-1406 (1973); Akerstrom et al., J. Immunol. 135:2589-2542 (1985)). The labeling agent can be modified with a detectable moiety, such as biotin, to which another molecule can specifically bind, such as streptavidin. A variety of detectable moieties are well known to those skilled in the art.

Commonly used assays include noncompetitive assays, *e.g.,* sandwich assays, and competitive assays. In competitive assays, the amount of polypeptide present in the sample is measured indirectly by measuring the amount of a known, added (exogenous) polypeptide of interest displaced (competed away) from an antibody that binds by the unknown polypeptide present in a sample. Commonly used assay formats include immunoblots, which are used to detect and quantify the presence of protein in a sample. Other assay formats include microarrays, similar to those described for nucleic acids above.

In some embodiments, controls for the disclosed methods include expression levels of the same biomarker genes as those tested in the patient in known tolerant individuals, healthy controls, or patients on standard immunosuppression. In some embodiments, the control can include expression levels of the biomarker genes from the same patient obtained at a different time, e.g., before kidney failure or transplantation. In some cases, the control can be expression of different genes, such as housekeeping genes for the appropriate cell or tissue type. The expression level of these genes is expected to remain relatively stable, allowing for relative comparison of biomarker expression level within and between individuals.

In some cases, a reference range for copy numbers of tolerance biomarker genes can be established through the use of reference control samples as well as reproducibility studies of the assay. Control reference samples can be run in each experiment, to control for variations in the rate of in vitro transcription prior to real time PCR. These reference samples can be created from a large pool of RNA from several patients. One or several rounds of comparisons can be run for validation purposes, and a reference RNA standard can be made to calibrate for assay runs, in particular differences between in vitro transcription efficiencies which in large part determines copy number. Additionally, the efficiency of the PCR reaction itself can be calibrated using a reference standard.

The assay can be standardized to determine an absolute copy number of the disclosed biomarker genes, deemed to be elevated in "tolerance" after calibration with reference standards, such that the absolute copy number can vary from assay to assay, but can be adjusted to be in the range of tolerance based on reference standards that allow determination of variation in the methodologies themselves. For example, original samples from which the data is gathered can be re-run multiple times with a single RNA reference sample to assess assay drift and variance. Once established, blind assessment of the same samples and/ or additional samples in a split duplicate manner can be done to calibrate a reference range. Such validation allows for more accurate assessment of positive or negative.

## VI. Databases and Computerized Methods of Analyzing Data

In some embodiments, the methods described herein are performed by a suitably programmed computer. The computer system for use with the methods described herein, as further described herein, is configured to accept and to process data and may be a single-processor or multiprocessor computer system. Examples of suitable computer systems include, but are not limited to, any one of various combinations of mainframe computers, minicomputers, personal computers, laptop computers, notebook computers, hand-held computers, personal digital assistants, mobile phones, set-top boxes, microprocessor-based consumer electronics, programmable consumer electronics, and the like. Additionally, the methods of the invention may be practiced on networked computers, CPU-clusters, workstations, and so-called mainframe computers. The computer system may be a locally accessed computer, a remotely accessed computer system (*e.g.* server), or a combination of both. Depending on the application and purpose, the computer system may have access or be accessible to the internet. It will be appreciated that the computer system may be a stand-alone system or a distributed system comprising multiple devices communicating with each other through a network. One of ordinary skill in the art will possess the necessary knowledge and skills for selecting, obtaining and utilizing a suitable computer system for practicing any aspect of the invention.

Various methods and formulae can implemented, in the form of computer program instructions, and executed on a computer as also described herein. Suitable programming languages for expressing the program instructions include, but are not limited to, one or more languages selected from the group consisting of: C, C++, an embodiment of FORTRAN such as FORTRAN77 or FORTRAN90, Java, Visual Basic, Perl, Tcl/Tk, JavaScript, and ADA. Various aspects of the methods can be written in different computing languages from one another, where such languages are preferred for particular applications, and the various aspects are caused to communicate with one another by appropriate system-level-tools available on a given computer. The computer program instructions are stored in a computer memory during execution, and can additionally be stored on any of various forms of computer-readable media known in the art, such as, but not limited to, CD-ROM, CD-R, $CD-RW_5$ flash memory, memory cards, memory sticks, DVD- ROM, USB-sticks, optical discs, or high capacity network storage drives. It is thus consistent with ordinary practice of the present invention that the computer program instructions can be delivered to a user on a transferable medium such as a CD-ROM, and also delivered over a computer network, such as by downloading over the Internet through a web- interface.

A database generated from the methods provided herein and the analyses described above can be included in, or associated with, a computer system for determining whether a kidney transplant recipient is tolerant. The database can include a plurality of digitally encoded "reference" (or "control") profiles. Each reference profile of the plurality can have a plurality of values, each value representing a level of a specific biomarker detected in a kidney transplant recipient. Alternatively, a reference profile can be derived from a individual that is normal. These profiles can be included in the database for consecutive or simultaneous comparison to a subject profile. The computer system can include a server containing a computer-executable code for receiving a profile of a subject and identifying from the database a matching reference profile that is diagnostically relevant to the subject profile. The identified profile can be supplied to a caregiver for diagnosis or further analysis.

Using standard programs, electronic medical records (EMR) can be accumulated to provide a database that combines biomarker data with additional information useful for determining tolerance. Patient information can be randomly assigned a numerical identifier to maintain anonymity. All data are can be stored on a network that provides access to multiple users from various geographic locations.

In some embodiments, the entire diagnosis or prognosis process can be accomplished using a computer. For example, a biological sample from a patient can be introduced to an gene expression detection device *(e.g.,* a microarray reader) functionally attached to the computer. The result can then be detected and recorded by the computer. In some embodiments, the result is then compared to a control value, *e.g.,* an average expression value for the given gene or set of genes.

**VII. Kits**

Kits for using the biomarkers of the invention are available for use in diagnostic, and research applications, e.g., as described above. The kits of the invention may comprise any or all of the reagents to perform the methods described herein. In the diagnostic and research applications such kits may include any or all of the following: assay reagents, buffers, nucleic acids that bind to at least one of the genomic regions or genes described herein, hybridization probes and/or primers, antibodies or other moieties that specifically bind to at least one of the polypeptides encoded by the genes described herein, etc.

In some embodiments, the kit can include a biomarker array that includes a plurality of the biomarkers identified below in Tables X and Z. In some embodiments, the kit can include a biomarker array that includes at least one of the biomarkers selected from CD20, CD3, FOXP3, and perforin. In some embodiments, the kit includes the biomarker panel in an array format, e.g., with the markers in patterned, identifiable positions on a chip. Such a kit will generally include at least some of the following components: control samples *(e.g.,* positive for the included biomarkers), detectable labels, and buffer solutions.

A kit can also comprise components for detecting RNA levels, *e.g.,* using RT-PCR. In such case, the kit can include primer and/ or probe sequences to amplify a plurality of the biomarkers listed in Tables X and Z. In some embodiments, the kit can include primer sequences and/ or probes to amplify at least one of the biomarkers selected from CD20, CD3, FOXP3, and perforin. The kit can also include enzymes and reagents for carrying out the RT and PCR reactions.

In some embodiments, the kit will include reagents for determining cell type, *e.g.,* using immunoassays such as flow cytometry (FACS) or fluorescence microscopy. Cell type can also be determined using nucleic acid or protein-based assays based on detection of cell-type specific genes. An immunoassay kit can include labeled antibodies for detecting markers expressed on the surface of various immune cells. The kit can also include staining buffers, wash reagents, and control samples. An exemplary kit designed for detecting B cell populations by FACS can include one or more of the following: labeled antibodies for CD19, CD24, CD27, CD38, IgM, IgD, and CD86; buffer mixes for staining, washing, and/ or fixing the cells; control samples; labware for use in FACS machines; directions for staining and recommended FACS settings, etc.

One of skill will appreciate that the components of the kit can be packaged and made available individually or in a single package.

In addition, the kits can include instructional materials containing directions *(i.e.,* protocols) for the practice of the methods of this invention. While the instructional materials typically comprise written or printed materials they are not limited to such. Any medium capable of storing such instructions and communicating them to an end user is contemplated by this invention. Such media include, but are not limited to electronic storage media *(e.g.,* magnetic discs, tapes, cartridges, chips), optical media *(e.g.,* CD ROM), and the like. Such media may include addresses to internet sites that provide such instructional materials. The kits can also include appropriate hardware and/ or software for detecting and analyzing the assay results.

**VIII. Examples**

**A. Summary**

Peripheral blood mononuclear cells (PBMCs) and whole blood total RNA from 25 tolerant kidney transplant recipients was studied. Tolerance was defined by cessation of all immunosuppression for at least one year and with maintenance of stable graft function. Gene expression profiles and peripheral blood subsets were compared with those of kidney transplant recipients who had stable graft function on immunosuppression, and normal healthy control participants.

## B. Materials and methods

Primary gene-expression profiling data are available from the Gene Expression Ominbus of the National Center for Biotechnology Information on the World Wide Web at ncbi.nlm.nih.gov/geo.

**Participant recruitment and study protocol:** One hundred adult renal transplant recipients and healthy volunteers were recruited nationwide at five participating centers between 2004 and 2007: Emory University (Atlanta, GA), the National Institutes of Health (Bethesda, DC), Swedish Medical Center (Seattle, WA), and the University of Wisconsin (Madison, WI). The protocol was approved by the IRB of each participating center, and by a DSMB convened by the National Institutes of Allergy and Infectious Diseases. Blood samples were collected by either standard phlebotomy (200 ml total volume) or leukapheresis.

Renal allograft recipients were enrolled into three groups: (1) Tolerant participants (TOL; N = 25), defined as individuals who, for at least one year prior to enrollment, had not taken immunosuppressive medications and had stable renal function, serum creatinine (Cr) within 25% of baseline (as evaluated by three experienced transplant physicians); (2) CAN (Chronic Allograft Nephropathy) participants, defined as those with impaired renal function (50% increase in their baseline Cr at time of enrollment relative to their initial post-transplant baseline) due to presumed immune mediated allograft rejection; (3) SI subjects (N = 34), with clinically stable renal function using the same criteria as TOL while on a maintenance triple drug immunosuppressive regimen (including a calcineurin or mTOR inhibitor, an anti-proliferative agent, and corticosteroids). An additional group of normal, healthy control (HC) participants with no known history of renal disease/dysfunction or evidence of acute medical illness was enrolled. Breakdown of samples used in each assay are shown in Table 1.

### Table1: Samples Analyzed by Assay

| Multi-assay biomarker identification | | | | | | |
|---|---|---|---|---|---|---|
| Group | Flow cytometry* (Fresh) | Peripheral Blood Microarray | Urine RT-PCR | Mass ARRAY | Frozen Flow ITN | IOT |
| TOL | 25 | 19 | 15 | 25 | 23 | 6 |
| SI | 30 | 27+ | 20 | 30 | 31 | 14 |
| HC | 42 | 12 | NA | 20 | 13 | 18 |

| Prediction of clinical state | | | | | |
|---|---|---|---|---|---|
| Group | Sequenom ITN TRN | ITN TST | Frozen Flow ITN | IOT | |
| TOL | 19 | 6 | 23 | 6 | |
| SI | 24 | 6 | 31 | 12 | **(Samples with complete data)** |

*Includes TOL-TRN, TOL-TST, SI-TRN, SI-TST
+Three samples run on Affymetrix microarray only, not on MassARRAY QGE

For the purposes of predictive modeling and verification of our initial findings, we divided the tolerant participants into a training set (TOL-TRN) of 19 and a test set (TOL-TST) of 6 participants based on enrollment prior to or after a reference date, respectively. The SI group was also divided into a training set (SI-TRN) of 27 and a test set (SI-TST) of 6 for modeling and verification (also based on enrollment timing).

We limited the comparison group to SI (stable patients doing well) instead of CAN patients based on the concept that this population is the most clinically relevant. SI individuals would be considered candidates for immunosuppressive minimization, while those with CAN likely would not.

Finally, frozen PBMC's were collected from our patient cohorts (ITN) and an independent set of kidney transplant recipients in Europe (IOT), and used for flow cytometry analysis. Frozen PBMC's from the ITN patients and 3 of the European cohorts, (TOL, HC, and SI (calcineurin inhibitors)), were studied by flow cytometry at the same time in an independent laboratory (see below).

**HLA Typing:** Whole blood from recipients and donors (when samples were available) was collected and frozen in cryotubes then shipped to a central laboratory (UCSF Immunogenetics and Transplantation Laboratory, San Francisco, CA) for automated nucleotide sequencing, which was performed from genomic DNA by selective amplification (PCR) of target exons from each locus for a particular allele. Loci sequenced included Class I HLA (HLA-A, -B and -C) and Class II HLA (HLA-DRB1/3/4/5, -DQA1 and -DQB1). Nucleotide sequencing was performed as previously described (Baxter-Lowe ASHI Laboratory Manual, 4th Edition, H Noreen (ed). 4 ed. American Society of Histocompatibility and Immunogenetics, 2002). When no donor samples were available, donor HLA types (serotypes) were obtained from United Organ Sharing Network (UNOS, Richmond, VA) database.

**HLA anti-donor crossmatching:** Initial screening for HLA antibodies on blinded samples was performed at a central laboratory (Emory University Histocompatibility Laboratory, Atlanta, GA) by flow cytometry using FlowPRA Screening™ beads (One Lambda, Inc., Canoga Park, CA). Antibody specificities of positive samples were determined using the LabScreen Single Antigen™ assay (One Lambda, Inc., Canoga Park, CA) (Gebel and Bray, Transplantation 2000; 69(7):1370-1374;Pei et al., Hum Immunol 1999; 60(12):1293-1302).

**Urine Quantitative RT-PCR:** RNAlater™ (Ambion, Austin, TX) was added to urinary cell pellets from urine samples (50-100 ml) centrifuged at room temperature (2000xg) for 30 min in order to extract total RNA. Samples were blinded and stored at -80°C prior to quantitative RT-PCR on 18 select genes (Granzyme B, Perforin, PI9, IL-4, Il-2, IL-10, interferon-gamma, CD3, CD20, CD25, CD103, FoxP3, CTLA4, TGF-beta, CTGF, IP10, MIG, and CXCR3) with 18S RNA used as a control and BK virus as previously described (Muthukumar et al., N Engl J Med 2005; 353(22):2342-2351).

**Flow Cytometry:** Whole blood was collected in 10 mL glass sodium heparin tubes (Becton Dickinson Vacutainer, Franklin Lakes, NJ) and shipped ambient overnight to the ITN Flow Cytometry Core (Roswell Park Cancer Institute, Buffalo, NY). Using a stain-lyse method, cells from blinded samples were stained with five-color monoclonal antibody panels conjugated to FITC, PE, PERCP, PECY7, or APC (Becton Dickinson, San Jose, CA) (Steward, Immunophenotyping. 1 ed. John Wiley and Sons, 2000). Marker/fluorophore combinations used are described in Table 2.

## Table 2: Flow Cytometry Panels

Antibody panels for whole blood staining:
1. FITC-CDIIc, PE-CD80, PerCP(CD3, 56, 19, 14), PeCy7 HLA-DR, APC-CD123;
2. FITC-CDIIc, PE-CD86, PerCP(CD3, 56, 19, 14), PeCy7 HLA-DR, APC-CD123;
3. FITC-CD45RA, PE-CD45RO, PerCP-CD8, PeCy7-CD4, APC-CD62L;
4. FITC-CD8, PE-CD25, PcrCP-CD4, PcCy7-CD3, APC-CD62L;
5. FITC-CDIc, PE-IgD, PerCP-C27, PeCy7-CD19, APC-IgM;
6. FITC-HLA-DR, PE-CD80, PerCP-C27, PeCy7-CD19, APC-CD86;
7. FITC-57, PE-57, PerCP-CD8 PeCy7-CD3, APC-CD14;
8. FITC-CD8, PE-CD69, PerCP-CD4 PeCy7-CD3, APC-HLA-DR;

Antibody panels for frozen cell staining:
1. FITC-IgD, PE-CDIc, PE-Alexa610-CD24, PE-Cy5-CD21, PerCP-Cy5.5-CD3, PE-Cy7-B220 PacificBlue-CD38, PacificOrange-Aqua live/dead, APC-CD27, APC-Cy7-CD19
2. FITC-V delta I, PE-V delta 2, PerCP gamma/delta, APC-CD3

For flow cytometry on frozen cells, PBMCs were stained in PBS/2 mM EDTA/0.5% BSA/5% normal mouse serum/5% normal rat serum on ice for 30 minutes with fluorochrome-conjugated mouse anti-human monoclonal antibodies as shown in Table 2. After washing with PBS/2 mM EDTA/0.5% BSA, cells were stained with LIVE/DEAD aqua-fluorescent reactive dye (Invitrogen) in PBS on ice for 30 minutes, and then fixed with 0.5% formaldehyde. Samples were blinded and run on a LSRII flow cytometer (BD Biosciences) at the University of Rochester.

For intracellular cytokine (IL-10 and TGFβ) evaluations, cells were divided into 2 samples and cultured in complete media (RPMI supplemented with 20% BSA) with brefeldin (1 μl/ml) and monensin (2 μM) in the presence or absence of stimulation (500 ng/ml PMA and 500 ng/ml ionomycin) for 5 hours. After culture, cells were washed with FACS Buffer (PBS plus 0.5% BSA) 2 x and then surface stained with extracellular antibody cocktail for 30 minutes at 4°C. Cells were then washed 2 x with PBS and stained with LIVE/DEAD Fixable Aqua Dead Cell Stain Kit (catalog no. L34957; Invitrogen) at 1:1,000 in PBS for 30 minutes at 4°C. Cells were then washed 2 x with FACS buffer and then fixed in 4% paraformaldehyde (Sigma-Aldrich) in PBS for 30 minutes at 4°C. Cells were subsequently permeabilized with Perm/Wash Buffer (catalog no. 554723; BD) for 30 minutes at 4°C. Cells with then intracellularly stained in Perm/Wash buffer with the ICS antibody cocktail for 1 hour at 4°C. Finally, cells were washed 2 x with Perm/Wash buffer and resuspended in 4% paraformaldehyde in PBS until analysis on the LSR II Flow Cytometer (BD) and with Flow Jo Software (Tree Star Inc.).

**Microarray:** Total RNA was obtained using the ABI Tempus™ whole blood collection system (Applied Biosystem, CA) and frozen for future processing. Total RNA was purified using methods as described (Asare et al., BMC Genomics 2008; 9:474). Total RNA can also be purified using a 6100 ABI Prep-Station (Applied Biosystem, Foster City, CA), with quality assessments using BioAnalyzer (Agilent, Santa Clara, CA) and the ABI© methods. Targets from blinded RNA samples were prepared and hybridized to Affymetrix HG-U133 2.0 Plus GeneChip® using the "GeneChip® Expression Analysis Technical Manual" (Affymetrix, Santa Clara, CA) with modifications as per Expression Analysis, Inc. (GeneChip Expression Analysis Technical Manual (2007) on the World Wide Web at expressionanalysis.com).

**MassARRAY Quantitative Gene Expression (QGE):** Multiplexed primer and competitive template designs were created using the MassARRAY QGE Assay Design software v1.0 (Sequenom, San Diego, CA) for random hexamer priming, such that at least one PCR primer spanned an exonic boundary per each transcript assayed. The genes tested were assayed in a series of 20-plex reactions on RNA from blinded samples and are shown in Table 3. Copy gene number determination was conducted as described in Asare et al., BMC Genomics 2008; 9:474.

**Table 3: Genes Included in QGE Panel**

| Ensemble Gene ID | HGNC gene symbol |
| --- | --- |
| ENSG00000197381 | ADARB1 |
| ENSG00000144218 | AFF3 |
| ENSG00000196139 | AKR1C3 |
| ENSG00000150347 | ARID5B |
| ENSG00000085224 | ATRX |
| ENSG00000112182 | BACH2 |
| ENSG00000112182 | BACH2 |
| ENSG00000114439 | BBX |
| ENSG00000119866 | BCL11A |
| ENSG00000119866 | BCL11A |
|  | BCL2L1 |
| ENSG00000023445 | BIRC3 |
| ENSG00000197299 | BLM |
| ENSG00000095585 | BLNK |
| ENSG00000153162 | BMP6 |
|  | BMP7 HUMAN |
| ENSG00000186265 | BTLA |
| ENSG00000186265 | BTLA |
| ENSG00000177364 | C9orf45 |
| ENSG00000129007 | CALML4 |
| ENSG00000115009 | CCL20 |
| ENSG00000117281 | CD160 |
| ENSG00000004468 | CD38 |
|  | CD3E |
|  | CD40 |
| ENSG00000196352 | CD55 |
| ENSG00000137101 | CD72 |
| ENSG00000007312 | CD79B |

(continued)

| Ensemble Gene ID | HGNC gene symbol |
|---|---|
| ENSG00000105810 | CDK6 |
| ENSG00000135837 | CEP350 |
|  | CTGF |
| ENSG00000149187 | CUGBP1 |
| ENSG00000163739 | CXCL1 |
| ENSG00000081041 | CXCL2 |
| ENSG00000163734 | CXCL3 |
| ENSG00000163737 | CXCL4 |
| ENSG00000185753 | CXorf38 |
| ENSG00000171604 | CXXC5 |
| ENSG00000138061 | CYP1B1 |
| ENSG00000085788 | DDHD2 |
| ENSG00000164821 | DEFA4 |
| ENSG00000135829 | DHX9 |
| ENSG00000197102 | DYHC |
| ENSG00000169508 | EBI2 |
|  | EDN1 |
| ENSG00000134001 | EIF2S1 |
| ENSG00000132507 | EIF5AP1 |
| ENSG00000158417 | EIF5B |
| ENSG00000187017 | ESPN |
| ENSG00000117525 | F3 |
| ENSG00000167483 | FAM129C |
| ENSG00000132185 | FCRLA |
| ENSG00000149557 | FEZ1 |
|  | FGF2 |
| ENST00000295713 | FNBP2 |
| ENSG00000150907 | FOXO1 |
| ENSG00000174804 | FZD4 |
| ENSG00000007237 | GAS7 |
| ENSG00000141098 | GFOD2 |
| ENSG00000088256 | GNA11 |
| ENSG00000179921 | GPBAR1 |
| ENSG00000159618 | GPR114 |
|  | HAVCR1 |
|  | HAVCR2 |
|  | HGF |
| ENSG00000204721 | HLA-C |

(continued)

| Ensemble Gene ID | HGNC gene symbol |
| --- | --- |
| ENSG00000204776 | HLA-C |
| ENSG00000204273 | HLA-DOB |
| ENSG00000090339 | ICAM1 |
|  | IFNG |
| ENSG00000073792 | IGF2BP2 |
| ENSG00000211895 | IGHA1 |
| ENSG00000211898 | IGHD |
| ENSG00000211893 | IGHG2 |
| ENSG00000211899 | IGHM |
| ENSG00000211956 | IGHV4-34 |
| ENSG00000132465 | IGJ |
| ENSG00000211592 | IGKC |
| ENSG00000211606 | IGKV1-12 |
| ENSG00000211613 | IGKV1-27 |
| ENSG00000211616 | IGKV1-33 |
| ENSG00000211630 | IGKV1D-13 |
| ENSG00000211612 | IGKV2-24 |
| ENSG00000211614 | IGKV2-28 |
| ENSG00000211607 | IGKV3-15 |
| ENSG00000211610 | IGKV3-20 |
| ENSG00000211598 | IGKV4-1 |
| ENSG00000211675 | IGLC1 |
| ENSG00000128322 | IGLL1 |
| ENSG00000206066 | IGLL3 |
| ENSG00000211648 | IGLV1-47 |
| ENSG00000211644 | IGLV1-51 |
| ENSG00000211666 | IGLV2-14 |
| ENSG00000161405 | IKZF3 |
| ENSG00000125538 | IL-1 |
|  | IL10 |
| ENSG00000110944 | IL23A |
|  | IL2RA |
|  | IL6 |
| ENSG00000169429 | IL8 |
| ENSG00000137265 | IRF-4 |
| ENSG00000162434 | JAK1 |
| ENSG00000123700 | KCNJ2 |
| ENSG00000091490 | KIAA0746 |

(continued)

| Ensemble Gene ID | HGNC gene symbol |
|---|---|
| ENSG00000111796 | KLRB1 |
| ENSG00000134545 | KLRC1 |
| ENSG00000205809 | KLRC2 |
| ENSG00000205810 | KLRC3 |
| ENSG00000134539 | KLRD1 |
| ENSG00000213809 | Klrk1 |
| ENSG00000136700 | KV105 HUMAN |
| | LOX |
| | LOXL1 |
| | LOXL2 |
| ENSG00000124831 | LRRFIP1 |
| ENSG00000197063 | MAFG |
| ENSG00000007264 | MATK |
| ENSG00000081189 | MEF2C |
| ENSG00000154035 | MGC33894 |
| ENSG00000101752 | MIB1 |
| ENSG00000091436 | MLTK HUMAN |
| ENSG00000102738 | MRPS31 |
| ENSG00000156738 | MS4A1 |
| ENSG00000156738 | MS4A1 |
| ENSG00000188895 | MSL-1 |
| ENSG00000122497 | NBPF16 |
| ENSG00000102908 | NFAT5 |
| ENSG00000100968 | NFATC4 |
| ENSG00000077150 | NFKB2 |
| ENSG00000183542 | NKG2F HUMAN |
| ENSG00000119508 | NR4A3 |
| ENSG00000148136 | OR13C4 |
| ENSG00000083454 | P2RX5 |
| ENSG00000157654 | PALM2 |
| ENSG00000081853 | PCDHGC5 |
| ENSG00000184588 | PDE4B |
| | PDGFA |
| | PECAM1 |
| ENSG00000078124 | PHCA |
| ENSG00000011422 | PLAUR |
| ENSG00000104164 | PLDN |
| | PLOD1 |

(continued)

| Ensemble Gene ID | HGNC gene symbol |
| --- | --- |
|  | PLOD2 |
|  | PLOD3 |
| ENSG00000140464 | PML |
| ENSG00000100413 | POLR3H |
| ENSG00000147535 | PPAPDC1B |
| ENSG00000147535 | PPAPDC1B |
| ENSG00000058272 | PPP1R12A |
| ENSG00000138814 | PPP3CA |
| ENSG00000185920 | PTCH1 |
| ENSG00000168229 | PTGDR |
| ENSG00000104388 | RAB2A |
| ENSG00000123892 | RAB38 |
| ENSG00000108774 | RAB5C |
| ENSG00000164754 | RAD21 |
| ENSG00000113522 | RAD50 |
| ENSG00000002016 | RAD52 |
| ENSG00000125249 | RAP2A |
| ENSG00000172575 | RASGRP1 |
| ENSG00000157110 | RBPMS |
| ENSG00000104856 | RELB |
| ENSG00000168421 | RHOH |
| ENSG00000067900 | ROCK1 |
| ENSG00000188846 | RPL14 |
| ENSG00000137154 | RPS6 |
| ENSG00000177409 | SAMD9L |
| ENSG00000124145 | SDC4 |
| ENSG00000187231 | SESTD |
| ENSG00000115524 | SF3B1 |
| ENSG00000145423 | SFRP2 |
| ENSG00000198574 | SH2D1B |
| ENSG00000182199 | SHMT2 |
| ENSG00000026751 | SLAMF7 |
| ENSG00000163848 | SLC12A8 |
| ENSG00000059804 | SLC2A3 |
|  | SMAD1 |
| ENSG00000175387 | SMAD2 |
|  | SMAD3 |
|  | SMAD5 |

(continued)

| Ensemble Gene ID | HGNC gene symbol |
|---|---|
|  | SMAD6 |
|  | SMAD7 |
| ENSG00000198742 | SMURF1 |
| ENSG00000159140 | SON |
| ENSG00000065526 | SPEN |
|  | SPG3A |
| ENSG00000142539 | SPIB |
| ENSG00000070182 | SPTB |
| ENSG00000035720 | STAP1 |
| ENSG00000165209 | STRBP |
| ENSG00000133789 | SWAP70 |
| ENSG00000166317 | SYNPO2L |
| ENSG00000149930 | TAOK2 |
| ENSG00000198211 | TUBB3 |
| ENSG00000131374 | TBC1D5 |
|  | TBX21 |
| ENSG00000100721 | TCL1A |
|  | TGFB1 |
|  | TGFB2 |
|  | TGFB3 |
|  | TGFBR1 |
|  | TGFBR2 |
| ENSG00000069702 | TGFBR3 |
|  | TGM2 |
| ENSG00000204490 | TNF |
| ENSG00000123610 | TNFAIP6 |
| ENSG00000076554 | TPD52 |
| ENSG00000164548 | TRA2A HUMAN |
| ENSG00000211829 | TRDC |
| ENSG00000106537 | TSPAN13 |
| ENSG00000140391 | TSPAN3 |
| ENSG00000168785 | TSPAN5 |
| ENSG00000137267 | TUBB2A |
| ENSG00000198211 | TUBB3 |
| ENSG00000104833 | TUBB4 |
| ENSG00000150991 | UBC |
| ENSG00000183696 | UPP1 |
| ENSG00000103194 | USP10 |

(continued)

| Ensemble Gene ID | HGNC gene symbol |
| --- | --- |
| ENSG00000115464 | USP34 |
| ENSG00000128218 | VPREB3 |
| ENSG00000156787 | WDR67 |
| ENSG00000108953 | YWHAE |
| ENSG00000205189 | ZBTB10 |
| ENSG00000185947 | ZNF267 |
| ENSG00000173276 | ZNF295 |
| ENSG00000130844 | ZNF331 |

**Data Analysis and Statistical Considerations:** Microarray data background adjustment, normalization, and summarization were performed using the Robust Multichip Average (RMA) method. Microarray quality assurance was carried out by detecting outlier arrays based on standard post-hybridization quality metrics (Asare et al., Bioinformatics 2009; 25(1):48-53). A linear mixed effect model was utilized for correcting potential processing batch effect as well as estimating clinical group effect. Pair-wise comparisons were performed with Tukey adjustment for group-level multiple comparisons, to identify differentially expressed genes between different clinical groups. Benjamini and Hochberg's approach for control of false discovery rate (FDR) was adopted for gene-wise multiple testing adjustments (Benjamini et al., Journal of the Royal Statistical Society 57[1], 289-300. 1995). Statistical criteria for identification of differentially expressed genes were FDR-adjusted p value < 0.05 and fold change > 1.5. In some circumstances, pathway analysis was performed using Ingenuity Pathway Analysis (Redwood City, CA).

In some cases, support vector machine (SVM), a modem machine learning approach was used to examine the participant group classification. Burges CJC. A Tutorial on Support Vector Machines for Pattern Recognition. Data Mining and Knowledge Discovery 2[2], 121-168. 1998. Ref Type: Journal (Full); Zhang et al. BMC Bioinformatics 2006; 7:197. In some cases, the procedures described in Furey et al. to partition data into balanced training and testing sets was utilized. In some instances, to reduce variability, the process of splitting into learning and testing sets was repeated 100 times and the misclassification rates averaged. Zhang et al. For feature selection the method described by Zhang et al. was used. Golub et al. Science 1999; 286(5439):531-537. The top 200 features were evaluated by considering both the misclassification rates and the limitation of follow-up biological investigations.

Urine RT-PCR data was normalized against 18S-rRNA; peripheral blood MassARRAY QGE was normalized against a set of five stable house-keeping genes. For urine RT-PCR, peripheral blood MassARRAY QGE, and flow cytometry data, a Shapiro-Wilk test was adopted to check whether data came from a normally distributed population. Although a log2 data transformation substantially reduced extent of deviation from the normal distribution, logarithm-transformed data of a substantial number of genes or cell populations still deviated from the normal distribution. Hence, a non-parametric Wilcoxon rank-sum test was conducted on the per gene basis for pair-wise comparisons between the clinical groups. Gene or cell population-wise multiple testing adjustments was performed using Benjamini and Hochberg's FDR method (Benjamini et al. 1995). A statistical criterion for identification of differentially expressed genes or cell populations was FDR-adjusted p value < 0.05. Hierarchical clustering images for microarray and MassARRAY QGE data were generated based on Pearson correlation using GeneSpring GX 7.3.1 (Agilent, Santa Clara). Box plot images were generated using S-Plus (TIBCO).

Classification approaches were applied to MassARRAY QGE and flow cytometry on frozen cells using log2 transformed data. In all instances, variable selection was performed using a 1-way ANOVA testing TOL vs. SI with multiple groups of variables ranging in size from one to 10 with a step of one. Linear Discriminant Analysis (LDA) with equal prior probabilities using leave one-out cross validation (LOOCV) was the most effective classification approach for MassARRAY QGE using three candidate genes (Pattern Classification and Scene Analysis. New York: John Wiley, 1973). The model was constructed using a training set of 19 TOL and 24 SI samples and then applied to an ITN test set of 6 TOL and 6 SI samples.

The normalized, $log_2$-transformed expression levels for the 1-3 gene signatures (IGKV1D-13, IGKV4-1, and IGLL1) were used to generate a probability score between 0 and 1 for each participant's membership in the TOL group using Equation 1, in which $\beta_i$ is the coefficient in the table, and $G_i$ is the expression level for each gene.

$$P_{Tol} = \frac{\exp(\beta_0 + \beta_1 G_{A+} \beta_2 G_{B+} \beta_3 G_C)}{1 + \exp(\beta_0 + \beta_1 G_{A+} \beta_2 G_{B+} \beta_3 G_C)}$$

(Equation 1)

Classification was based on the posterior probabilities with a 0.5 cutoff; thus, participants with a score of 0.5 or higher would be assigned to the TOL group. LOOCV was applied to determine how accurately the learning algorithm predicted data that it was not trained on. In using this approach, the LDA model was trained multiple times, using all but 1 of the training set data points. The sample that was removed was retested iteratively to generate the best PPV and NPV during training. Feature selection was embedded within the LOOCV process.

Support Vector Machine was the most effective for flow cytometry data in determining one population to have the best positive predictive value (PPV). The SVM model was configured with cost-based shrinking from one to 1001 with step 100 with a tolerance from 0.001. The kernel used a radial basis function (γ) set at 1/number of variables and leave one out cross validation (LIBSVM: a library for support vector machines. 2001). The model was constructed using 23 ITN TOL and 31 ITN SI samples and applied to the test set of European/INDICES OF TOLERANCE group samples consisting of 6 TOL and 12 SI samples. Partek Genomics Suite V 6.4 (Partek, St. Louis) was used for prediction approaches that allowed us to test over 1000 models using various feature combinations and SVM permutations.

## C. Results

### Example 1: Clinical features of study populations

Participants in the TOL and SI groups had excellent renal function even though TOL participants had ceased taking immunosuppressive medications for at least 1 year. Ages, genders and primary diseases leading to renal failure were similar between the two groups (Table 4).

| Table 4 | Tolerant Training | Tolerant Test | Standard Immunosuppression |
|---|---|---|---|
| **Donor Age in Years median (range)** | 49 (41-70) | 56.5 (47-66) | 52.5 (31-69) |
| **Donor Type** | 4 Cadaveric | 1 Cadaveric | 8 Cadaveric |
| | 14 Living (related) | 3 Living (related) | 14 Living (related) |
| | 1 Data Missing | 1 Living (unrelated) | 9 Living (unrelated) |
| | | 1 Data Missing | 2 Data Missing |
| **Gender** | 12 Male | 3 Male | 18 Male |
| | 7 Female | 3 Female | 15 Female |
| **Race** | 18 White | 6 White | 28 White |
| | 1 Asian | | 4 Black or African American |
| | | | 1 Asian |
| **Recipient Age in Years (median and range)** | 51 (27-77) | 51 (42-63) | 44 (26-75) |
| **Primary Cause of Renal Failure (number of individuals)** | 10.5% Post-streptococcal Glomerulonephritis (2) | 16.7% Cystic/ Polycystic Kidney Disease (1) | 12.1 % Hypertension (4) |
| | 15.8% Obstructive/ Reflux Nephrophathy (3) | 16.7% Diabetes Mellitus (1) | 12.1% IgA Nephrophathy (4) |
| | | 16.7% IgA Nephrophathy (1) | 24.2% Glomerulonephritis (8) |
| | 36.8% Glomerulonephritis (7) | | |
| | | 33.3% Glomerulonephritis (2) | 3% Diabetes Mellitus (1) |

(continued)

| Table 4 | Tolerant Training | Tolerant Test | Standard Immunosuppression |
|---|---|---|---|
| | 5.3% Cystic/ Polycystic Kidney Disease (1) | 16.7% Data Missing (1) | 3% Pyelonephritis/ Interstitial Nephritis (1) |
| | 5.3% Diabetes Mellitus (1) | | 6.1% Obstructive/ Reflux |
| | 5.3 % IgA Nephrophathy (1) | | Nephrophathy (2) |
| | 5.3% Pyelonephritis/ Interstitial Nephritis (1) | | 6/1% Post-streptococcal Glomerulonephritis (2) |
| | 15.8% Data Missing (3) | | 9.1 % Cystic/ Polycystic Kidney Disease (3) |
| | | | 9.1% Systemic Lupus Erythematosus (3) |
| | | | 15.2% Data Missing (5) |
| Cr Level median (range) | 1 (0.6-9.8) | 0.95 (0.7-1.5) | 1.4 (0.7-2.8) |
| Years Between Transplant and Enrollment median (range) | 20.42 (7-40.25) | 11.79 (5-19.67) | 5.5 (1-41.17) |
| HLA Mismatch | 0.92± 1.73 out of 6 | 0.67± 1.15 out of 6 | 3.58± 1.86 out of 6 |
| Time (years) of Cessation of Immunosuppressionmedian (range) | 13 (1-32) | 5.5 (2-22) | |
| Reason for Withdrawal of Immunosuppression | 12.5% Medical condition (2) | 100% Non-compliance (6) | |
| | 87.5% Non-compliance (14) | | |

Twelve of 25 participants in the TOL group could be tested for detectable anti-donor HLA antibodies. Primary donor HLA information was not available for the remainder of the tolerant cohort. Only 1 of the tolerant participants had detectable anti-donor antibodies, while in the SI group, 3 had donor specific antibodies. Participants in the TOL group were more matched for HLA than the SI group as shown in Table 1. While the majority of our TOL group was HLA matched, 5 of our participants were not matched for any HLA antigens. Time from transplant to enrollment was significantly higher in the tolerant group, while the number of patients with cadaveric donors was significantly more in the SI group. There were no other significant differences between the groups noted based on the clinical assessments in this study.

**Example 2: B-cell gene signatures distinguish tolerant participants from stable participants on continued immunosuppression**

TOL and SI groups were divided into two sets each. Tolerant patients were divided into a training set (TOL-TRN, N=19) and a test set (TOL-TST, N=6) based on their time of enrollment. Similarly, the SI cohort was divided into a training set (SI-TRN, N=27) and a test set (SI-TST, N=6). For microarray analyses, only samples from the TOL-TRN and SI-TRN were used. Affymetrix Genechips® were used to detect expressed gene profiles of whole blood total RNA from subjects in the TOL and SI groups. Statistical analysis for differentially expressed genes between these two groups was performed and the top differentially expressed genes were ranked in order, based on their mean fold change differences.

Five unique genes (TUBB2A, TCL1A, BRDG1, HTPAP and PPAPDC1B) reached statistical significance after a false discovery rate (FDR) correction was applied to the data. Both TCL1A and BRDG1 are B-cell specific. Of the 30 genes found to have a 2-fold increase in expression in the TOL-TRN group vs. the SI-TRN group, 22 were B-cell specific (see Figure 1 and Table X). Many of these genes are involved in B-cell activation and differentiation, including genes encoding immunoglobulin heavy, light and joining chains, and HLA (Hoffmann et al., J Leukoc Biol 2003; 74(4):602-610; Matthias et al., Nat Rev Immunol 2005; 5(6):497-508). In contrast, few genes were observed to be more highly expressed in the SI-TRN group. One of these, Tubulin 2A (TUBB2A) was highly differentially expressed (7-fold difference). Over-expression of this gene may be due to prolonged use of calcineurin inhibitors, which have been shown to induce tubulin expression (Cui et al., Neuroscience 2007; 146(3):986-999).

Comparison of whole blood total RNA from TOL-TRN and HC subjects revealed no significant differences in expression levels. The similarity in profiles between TOL-TRN and HC groups is illustrated using hierarchical clustering of the 30 genes that were differentially expressed between TOL-TRN and SI-TRN (Figure 1).

**Table X: Microarray genes with at least 2-fold higher expression in TOL than SI**

| Gene Name | Common | Genbank | Affymetrix Gene Symbol | Description |
|---|---|---|---|---|
| 220059_at | BRDG1 | N_012108 | BRDG1 | BCR downstream signaling 1 |
| 220068_at | VPREB3 | N_013378 | VPREB3 | pre-B lymphocyte gene 3 |
| 221671_x_at | | M63438 | --- | Immunoglobulin kappa light chain mRNA, partial cds |
| 226150_at | HTPAP | BF111651 | HTPAP | HTPAP protein |
| 216576_x_at | | AF103529 | --- | Clone H10 anti-HLA-A2/A28 immunoglobulin light chain variable region mRNA, partial cds |
| 217022_s_at | MGC27165 | S55735 | MGC27165 | hypothetical protein MGC27165 |
| 217148_x_at | IGLJ3 | AJ249377 | IGLJ3 | immunoglobulin lambda joining 3 |
| 230983_at | BCNP1 | BE646461 | BCNP1 | B-cell novel protein 1 |
| 234764_x_at | IGL@ | U96394 | --- | Clone P2-147 anti-oxidized LDL immunoglobulin light chain Fab mRNA, partial cds |
| 235372_at | FREB | AW575245 | FREB | Fc receptor homolog expressed in B cells |
| 243968_x_at | FCRH1 | AI572979 | FCRH1 | tn64g01.x1 NCI_CGAP_Lym12 Homo sapiens cDNA clone IMAGE: 2174352 3' similar to gb:L19593 HIGH AFFINITY INTERLEUKIN-8 RECEPTOR B (HUMAN);, mRNA sequence. |
| 217979_at | TM4SF13 | N_014399 | TM4SF13 | transmembrane 4 superfamily member 13 |
| 227198_at | LAF4 | AW085505 | LAF4 | lymphoid nuclear protein related to AF4 |
| 229513_at | STRBP | AK025613 | STRBP | spermatid perinuclear RNA binding protein |
| 230877_at | IGHM | AI492643 | IGHM | immunoglobulin heavy constant mu |
| 209995_s_at | TCL1A | BC003574 | TCL1A | T-cell leukemia/lymphoma 1A |
| 211430_s_at | IGHG1 | M87789 | IGHG3 | |
| 211644_x_at | | L14458 | --- | Immunoglobufin kappa light chain mRNA, partial cds |
| 212592_at | IGJ | AV733266 | IGJ | immunoglobulin J polypeptide, linker protein for immunoglobulin alpha and mu polypeptides |
| 205671_s_at | HLA-DOB | N_002120 | HLA-DOB | major histocompatibility complex, class II, DO beta |
| 207655_s_at | BLNK | N_013314 | BLNK | B-cell linker |
| 209138_x_at | IGLJ3 | M87790 | IGL@ | |

(continued)

| Gene Name | Common | Genbank | Affymetrix Gene Symbol | Description |
|---|---|---|---|---|
| 214916_x_at | IGHM | BG340548 | --- | Partial mRNA for IgM immunoglobulin heavy chain variable region (IGHV gene), clone LIBPM376 |
| 215121_x_at | | AA680302 | --- | CDNA FLJ26905 fis, clone RCT01427, highly similar to Ig lambda chain C regions |
| 215379_x_at | IGLJ3 | AV698647 | IGLJ3 | immunoglobulin lambda joining 3 |
| 215925_s_at | CD72 | AF283777 | CD72 | CD72 antigen |
| 209160_at | AKR1C3 | AB018580 | AKR1C3 | aldo-keto reductase family 1, member C3 (3-alpha hydroxysteroid dehydrogenase, type II) |
| 212827_at | IGHM | X17115 | IGHM | unnamed protein product; precursor (AA -15 to 612); Human mRNA for IgM heavy chain complete sequence. |
| 214677_x_at | IGLJ3 | X57812 | IGLJ3 | immunoglobulin lambda joining 3 |
| 214836_x_at | | BG536224 | --- | Clone 2-12 immunoglobulin light chain mRNA, partial cds |

Further evidence for B-cell involvement in tolerance is supported by the gene expression profiles of urinary cell sediments. Of the 18 genes tested by quantitative real-time PCR, only the CD20 transcript was significantly higher in TOL than SI participants (Figure 2). FOXP3 and CD20 expression was significantly lower in urine sedimentary cells from HC compared to TOL-TRN (Figure 2A and B). CD3 and Perforin expression levels were also significantly lower in HC compared to TOL-TRN (Figure 2C and D). This is consistent with healthy subjects having few to no lymphocytes in their urine. Urine from 7 of the 25 HC samples did not have sufficient RNA because of the low numbers of urine sedimentary cells, and these were excluded from the analysis. These results provide another unique tool for predicting tolerance in kidney transplant recipients.

**Example 3: Multiplex real time PCR confirms B-cell gene signature and identifies three genes which predict tolerance**

MassARRAY QGE was performed on all TOL, SI and HC participants to develop a more quantitative approach for defining tolerance-specific expressed gene profiles and to support our microarray findings. Probe-primer sets for 228 genes were made for the MassARRAY QGE which was run on all participants (Table 3). Clustering of the MassARRAY QGE data (Figure 3), revealed 31 unique genes that were statistically significantly different between the TOL-TRN and SI-TRN groups (Figure 3 and Table 5). P value adjustments (p values < 0.05) were made using false discovery rate (FDR) correction to account for multiple comparisons. Using the FDR correction, no genes were significantly different between TOL and HC. However, observed differences without the FDR correction are disclosed in Table 5.

Table 5: Genes differentially expressed ranked by p-value.

| TOL vs. SI | TOL vs. HC |
|---|---|
| Gene | Gene |
| | |
| IGKVID-13 | IGKVID-13 |
| FCRLA | IGKVI-33 |
| IGHM | IGF2BP2 |
| IGHV4-34 | VPREB3 |
| IGKC | BCL2LI |
| IGKV3-20 | TUBB2A |
| IGKV4-1 | RAB2A |
| FAM129C | KirkI |
| MS4AI | IL-1 |
| MS4AI_2 | SF3BI |
| KVI05_HUMAN | PTCHI |
| IGKVI-12 | PPPIRI2A |
| IGJ | CXCL4 |
| IKZF3 | NBFP16 |
| IGLLI | IGHG2 |
| IGLCI | TSPAN5 |

| | |
|---|---|
| BIRC3 | TSPAN13 |
| IGHAI | GFOD2 |
| IGHD | MLTK_HUMAN |
| PPAPDCIB | TGFBR2 |
| IRF-4 | CD40 |
| STAPI | |
| EBI2 | |
| IGKV2-28 | |
| BTLA_2 | |
| HLA-C | |
| IGKV3-15 | |
| TUBB2A | |
| ARID5B | |
| IGKVI-27 | |
| PLOD3 | |

Of the 31 differentially expressed biomarkers in TOL-TRN vs. SI-TRN, 17 were originally detected by microarray. None of the genes found to be involved in liver transplant tolerance by Llordella-Martinez were differentially expressed between our TOL and SI cohorts. Again, most of the genes over-expressed in the tolerant cohort were B cell specific (26/31), with many encoding $\kappa/\lambda$ light chains of immunoglobulin. Finally, 23 were differentially expressed between the TOL-TRN and the CAN groups ($p < 0.05$). The 31 genes are shown below in Table Z.

**Table Z: Sequenom MassARRAY genes with higher expression in TOL patients**

| Gene Name | Overlap With 30-Gene Signature | Prediction Genes | |
|---|---|---|---|
| IGKV4-1 | | X | |
| IGLC1 | | | |
| IGLL1 | | X | |
| IKZF3 | | | |
| IGKV1D-13 | | X | |
| IGKV2-28 | | | |
| IGKV3-15 | | | |
| IGKV3-20 | | | |
| PLOD3 | | | |
| PPAPDC1B | | | |
| STAP1 | | | |
| TUBB2A | | | |
| IRF-4 | | | |
| KV105 HUMAN | | | |
| MS4A1 | X | | |
| FAM129C | | | |
| FCRLA | | | |
| HLA-C | | | |
| IGHA1 | | | |
| ARID5B | | | |
| BIRC3 | | | |
| BTLA 2 | | | |
| EBI2 | | | |
| IGKC | X | | |
| IGKV1-12 | | | |
| IGKV1-27 | | | |
| MS4A1_2 | | | |
| IGHD | | | |
| IGHM | X | | |
| IGHV4-34 | | | |
| IGJ | X | | |

Using the 228 MassARRAY QGE genes, we performed feature selection and applied SVM and linear discriminant analysis (LDA) prediction approaches and found LDA to define a smaller set of genes that would be highly predictive for tolerance based on TOL-TRN and SI-TRN participants. SI participants were using standard triple drug immunosuppression. In this model, 3 genes were found to be predictive, giving a positive predictive value (PPV) of 83% and a negative predictive value (NPV) of 84% (Table 6).

### Table 6: MassARRAY QGE – ITN Training Set

| Confusion Matrix | | | | |
|---|---|---|---|---|
| | | **Actual** | | |
| | | TOL-TRN | SI-TRN | |
| **Predicted** | TOL-TRN | 15 | 3 | PPV = 83% |
| | SI-TRN | 4 | 21 | NPV = 84% |

Variable Name:IGKVID-13; IGKV4-1; IGLLI

To directly test the predictive value of these 3 genes, we used the cohort of 6 TOL and 6 SI patients, termed TOL-TST and SI-TST. Six of 6 TOL-TST participants were correctly identified as tolerant and 5 of 6 correctly identified as SI yielding a PPV=86% and an NPV = 100% (Table 7).

### Table 7: MassARRAY QGE – ITN Test Set

| Confusion Matrix | | | | |
|---|---|---|---|---|
| | | **Actual** | | |
| | | TOL-TST | SI-TST | |
| **Predicted** | TOL-TST | 6 | 1 | PPV = 86% |
| | SI-TST | 0 | 5 | NPV = 100% |

Variable Name:IGKVID-13; IGKV4-1; IGLLI

These genes are IGKV4-1, IGLL1 and IGKV1D-13, and their expression levels for each patient are plotted in Figures 4 and 5. As shown, these genes clearly separate TOL from SI patients for the majority of participants in the training set, and for all but one SI participant in the test set. Figure 5 indicates mRNA copy numbers for each of these genes. All three of these genes encode $\kappa$ or $\lambda$ light chains, which are up-regulated during the transition of pre-B to mature B cells and during class switch and receptor editing that occurs following stimulation of mature B cells with antigen.

We also determined the predictive value using just one or two of the three biomarkers: IGKV1D-13, or IGKV1D-13 + IGKV4-1.

The results show that expression of the single biomarker IGKV1D-13 is quite predictive of tolerance. LDA data for the training and test sets are shown in Tables 8 and 9. For the training set, high levels of IGKV1D-13 were predictive of 22 of 24 cases, and low levels were predictive for 15 of 19 cases, resulting in an average percent correct of 86%.

### Table 8: Training set based on IGKV1D-13 expression

| Confusion Matrix | | | | |
|---|---|---|---|---|
| | | **Actual** | | |
| | | TOL-TRN | SI-TRN | |
| **Predicted** | TOL-TRN | 15 | 2 | PPV = 88% |
| | SI-TRN | 4 | 22 | NPV = 85% |

For the test set, high levels of IGKV1D-13 were predictive in 5 of 6 cases, and low levels were predictive in 6 of 6 cases, leading to an average % correct of 91.5%

### Table 9: Test set based on IGKV1D-13 expression

| Confusion Matrix | |
|---|---|
| | **Actual** |

| | | TOL-TST | SI-TST | |
|---|---|---|---|---|
| **Predicted** | TOL-TST | 6 | 1 | PPV = 86% |
| | SI-TST | 0 | 5 | NPV = 100% |

Data for the two-gene biomarker set is shown below in Tables 10 and 11. The positive and negative predictive value of tolerance using IGKV1D-13 and IGKV4-1 as biomarkers are also quite high. The average percent correct was 83.7% for the training set and 91.5% for the test set.

### Table 10: Training set based on IGKV1D-13 and IGKV4-1 expression

| Confusion Matrix | | | | |
|---|---|---|---|---|
| | | **Actual** | | |
| | | TOL-TRN | SI-TRN | |
| **Predicted** | TOL-TRN | 15 | 3 | PPV = 83% |
| | SI-TRN | 4 | 21 | NPV = 84% |

### Table 11: Test set based on IGKV1D-13 and IGKV4-1 expression

| Confusion Matrix | | | | |
|---|---|---|---|---|
| | | **Actual** | | |
| | | TOL-TST | SI-TST | |
| **Predicted** | TOL-TST | 6 | 1 | PPV = 86% |
| | SI-TST | 0 | 5 | NPV = 100% |

Example 4: Flow cytometry of whole blood revealed increased numbers of naïve B cells in tolerant participants.

Flow cytometric studies of whole blood revealed a significant difference between the TOL and SI groups in the number of total B cells (CD19+) and naive B cells (CD19+/CD27-/IgM+/IgD+) (Figure 6A and B). The mean B cell number of the TOL cohort was significantly greater than in the SI group (P < 0.01), but did not reach significance compared with the HC cohort (P = 0.10). However, there were significant differences between the TOL cohort and both the SI and HC group with respect to naive B cell numbers (P $\leq$ 0.05, Figure 6B). In addition, $CD86^+CD19^+$ B cells and memory B cells ($CD19^+CD27^+IgM^+IgD^+$) were significantly different between the TOL and HC cohorts (P < 0.01 and P = 0.03, respectively, Figure 6C and D).

Many subpopulations of lymphocytes were measured in our analyses (Table 2), and we did find other lymphocyte subpopulations that were significantly different between the TOL and SI or TOL and HC groups. These included HLA-$DR^+CD4^+$ T cells and NK cells, among others. Of those that were significantly different, we highlight here the B cells and B cell subsets, as these differences correlated with our findings using microarrays and PCR.

Statistical analyses of total white blood cell count means for each group demonstrated that the observed group-specific differences were not due to overall changes in the number of total white blood cells. Mean WBC counts for the TOL, SI, and HC groups were 6.7, 8.1 and 5.8 thousand cells/$\mu$l, respectively. Furthermore, the observation that selected populations of B cells distinguish tolerant kidney transplant recipients from healthy controls, suggests that the B cell signature associated with tolerance is not simply a consequence of immunosuppression.

Finally, we evaluated the B-cell signature in an independent set of kidney transplant recipient patients enrolled in the Indices of Tolerance (IOT) study of tolerant renal transplant recipients. These studies were performed with additional surface markers to be able to differentiate between naive and transitional cells and discriminate transitional subsets. Frozen PBMC's from our (ITN) patients and three of the Indices of Tolerance cohorts (tolerant immunosuppressive drug-free recipients, transplant recipients receiving a calcineurin-based immunosuppressive regimen with stable function, and healthy control subjects) were studied by flow cytometry.

The percentage of total B cells (CD19+) and naive B cells (CD19+/CD27-/IgD+) in the lymphocyte gate was higher in each TOL group when compared with their respective SI group in both our and IOT samples (Figure 7A and B). Lower numbers of naive B cells were observed in all groups of samples provided by the IOT collaborative relative to the ITN samples. Differences in freezing methods may have caused these shifts. Importantly, the patients in the IOT tolerant cohort were not as well HLA-matched with their donors as were those in the ITN tolerant cohort, suggesting that the increased B-cell numbers were not a direct result of HLA matching.

Transitional B cells (CD19+/CD38+/CD24+/IgD+) were examined in these samples. These cells were of interest because of our gene signature and the regulatory role that has been proposed in murine models for cells with a similar immature phenotype (Evans et al., J Immunol., 178(12):7868-78 (2007)). In both IOT and ITN samples, there were increased numbers of transitional B cells in the TOL versus SI group comparisons (Figure 7C). Linear discriminant analyses of the frozen flow assays, also showed these cells to be the most predictive, with a PPV of 96% and NPV of 87% in our samples and a PPV of 63% and NPV of 90% in the European IOT samples that served as the test set (sensitivity 83%, specificity 75%) (Tables 12 and 13).

### Table 12: Flow Cytometry – ITN Training Set

| Confusion Matrix | | | | |
|---|---|---|---|---|
| | | **Actual** | | |
| | | TOL-ITN | SI-ITN | |
| **Predicted** | TOL-ITN | 22 | 4 | PPV = 85% |
| | SI-ITN | 1 | 27 | NPV = 96% |

Variable Name: Transitional B Cells

### Table 13: Flow Cytometry – IOT Test Set

| Confusion Matrix | | | | |
|---|---|---|---|---|
| | | **Actual** | | |
| | | TOL-IOT | SI-IOT | |
| **Predicted** | TOL-IOT | 5 | 3 | PPV = 63% |
| | SI-IOT | 1 | 9 | NPV = 90% |

Variable Name: Transitional B Cells

**Example 5: Elevated expression of intracellular IL-10 in TOL patients**

We sought next to determine whether the increased B cells and B cell sub-populations in TOL and HC patients produce immunomodulatory cytokines, such as IL-10 or TGFβ, that might be implicated in tolerance (Fillatreau et al., Nat Rev Immunol., 8(5):391-397 (2008)). Intracellular cytokine staining was performed on frozen PBMCs from 21 TOL, 32 SI, and 13 HC patients. The cells were studied both unstimulated and cultured in the presence of PMA plus ionomycin for 5 hours. Total B cells and 6 subsets, including T1 and T2 transitional B cells ($CD38^+CD24^+$), switched memory ($CD27^+IgD^-$), $CD27^-$ memory ($CD27^-IgD^-$), naive and T3 transitional ($CD27^-IgD^+$), unswitched memory ($CD27^+IgD^+$), and plasmablasts ($CD27^+CD38^+IgD^-$), were analyzed for IL-10 and TGFβ intracellular staining.

We observed a statistically significant increase in T1 plus T2 transitional B cells expressing IL-10 in the TOL group relative to the SI or HC groups (Figure 8A). The result must be interpreted cautiously, as the overall percentages were extremely low, with many samples containing no IL-10-producing cells, and with a large overlap in the range of the groups. Stimulation conditions that were limited in length and intensity in order to maintain the cell surface phenotype and thus determine IL-10 production within different B cell subpopulations. No differences in the number of TGFβ-expressing B cells or subsets between cells from TOL or any other group were found (Figure 8B).

**Example 6: Assessment of frequency of B-cell signature among kidney transplant recipients**

Peripheral blood is obtained from approximately 300 patients and the B-cell signature is assessed. From this, we can estimate the frequency of the signature among stable kidney transplant recipients (i.e., those with stable graft function on immunosuppressive therapy). Based on preliminary data, the rate of kidney transplant recipients who express the B cell signature should be approximately 1 in 10 or 10%.

Once frequency is established in the general stable kidney transplant population, prospective minimization and withdrawal studies are pursued to assess the predictive value of the B cell tolerance signature. Based on early data, the B-cell signature predicts those patients amenable to immunosuppression minimization and/ or withdrawal.

It is understood that the examples and embodiments described herein are for illustrative purposes only and that various modifications or changes in light thereof will be suggested to persons skilled in the art and are to be included within the spirit and purview of this application and scope of the appended claims. All publications, patents, and patent applications cited herein are hereby incorporated by reference in their entirety for all purposes.

**Aspects of the disclosure**

1. A biomarker array comprising at least one biomarker selected from the group consisting of the biomarkers disclosed in Table X and Table Z.

2. The biomarker array of aspect 1, comprising IgκV1D-13.

3. The biomarker array of aspect 1, comprising IgκV4-1 and IgκV1D-13.

4. The biomarker array of aspect 1, comprising IgκV4-1, IgλL1, and IgκV1D-13.

5. The biomarker array of aspect 1 comprising at least 10 biomarkers selected from the group consisting of the biomarkers disclosed in Table X and Table Z.

6. The biomarker array of aspect 1 comprising at least 10 biomarkers selected from the group consisting of the biomarkers disclosed in Table Z.

7. The biomarker array of aspect 1, wherein the biomarker array does not comprise MS4A1.

8. A method for predicting tolerance in a transplant recipient for the transplant, the method comprising, (i) detecting in a biological sample from the transplant recipient the expression level of at least one biomarker selected from the group consisting of the biomarkers disclosed in Table X and Table Z; and (ii) comparing the expression level of the at least one biomarker with the expression level of the same at least one biomarker from a biomarker control, thereby predicting the tolerance of the transplant recipient for the transplant.

9. The method of aspect 8, wherein expression of IgκV1D-13 is detected and compared.

10. The method of aspect 8, wherein expression of IgκV4-1 and IgoV1D-13 is detected and compared.

11. The method of aspect 8, wherein expression of IgκV4-1, IgλL1, and IgκV1 D-13 is detected and compared.

12. The method of aspect 8, wherein the biomarker control is selected from the group consisting of: an individual non-tolerant transplant recipient; a group of non-tolerant transplant recipients; an individual healthy non-transplant recipient; a group of healthy non-transplant recipients; a sample from the transplant recipient taken at a different time.

13. The method of aspect 12, wherein the biomarker control is an individual or group of non-tolerant transplant recipients and expression level of the at least one biomarker is increased at least two-fold over the control.

14. The method of aspect 8, wherein said detection step comprises using a nucleic acid microarray or a PCR-based method.

15. The method of aspect 8, wherein expression of at least 10 biomarkers selected from the group consisting of the biomarkers disclosed in Table X and Table Z is detected and compared.

16. The method of aspect 8, further comprising adjusting the course of immunosuppressive therapy for the transplant recipient depending on whether the transplant recipient is predicted to be tolerant of the transplant.

17. A method of determining a course of immunosuppressive therapy for a transplant recipient, said method comprising: (i) detecting in a biological sample from the transplant recipient the expression level of at least one biomarker selected from the group consisting of the biomarkers disclosed in Table X and Table Z; (ii) comparing the expression level of the at least one biomarker with the expression levels of the same at least one biomarker from a biomarker control; (iii) predicting whether the transplant recipient will be tolerant of the transplant based on the comparison in step (ii); and (iv) adjusting the course of immunosuppressive therapy for the transplant recipient depending on whether the transplant recipient is predicted to be tolerant of the transplant.

18. The method of aspect 17, wherein expression of IgκV1D-13 is detected and compared.

19. The method of aspect 17, wherein expression of IgκV4-1 and IgκV1D-13 is detected and compared.

20. The method of aspect 17, wherein expression of IgκV4-1, IgλL1, and IgκV1D-13 is detected and compared.

21. The method of aspect 17, wherein the biomarker control is selected from the group consisting of: an individual non-tolerant transplant recipient; a group of non-tolerant transplant recipients; an individual healthy non-transplant recipient; a group of healthy non-transplant recipients; a sample from the transplant recipient taken at a different time.

22. The method of aspect 21, wherein the biomarker control is an individual or group of non-tolerant transplant recipients and expression level of the at least one biomarker is increased at least two-fold over the control.

23. The method of aspect 17, wherein said method is carried out once immunosuppressive therapy has been initiated.

24. The method of aspect 17, wherein said method is carried out more than once.

25. The method of aspect 17, wherein immunosuppressive therapy is reduced if the transplant recipient is predicted to be tolerant of the transplant.

26. The method of aspect 17, wherein no immunosuppressive therapy is prescribed if the transplant recipient is predicted to be tolerant of the transplant.

27. A method for predicting tolerance in a transplant recipient for the transplant, the method comprising, detecting in a biological sample from the transplant recipient the amount of at least one B cell population in the sample; and comparing the amount of said at least one B cell population to the amount of the same at least one B cell population in a biological sample from a B cell control, wherein the at least one B cell population is selected from: total B cells, naive B cells, memory B cells, transitional B cells, and CD86+ B cells, thereby predicting the tolerance of the transplant recipient for the transplant.

28. The method of aspect 27, wherein the detecting comprises fluorescence activated cell sorting (FACS).

29. The method of aspect 27, wherein the B cell control is selected from the group consisting of: an individual non-tolerant transplant recipient; a group of non-tolerant transplant recipients; an individual healthy non-transplant recipient; a group of healthy non-transplant recipients; a sample from the transplant recipient taken at a different time.

## Claims

1. A method for predicting tolerance in a transplant recipient for the transplant, the method comprising,

      (i) detecting in a biological sample from the transplant recipient the expression level of IgκV1D-13; and
      (ii) comparing the expression level of IgκV1D-13 with the expression level of a biomarker control,
      thereby predicting the tolerance of the transplant recipient for the transplant.

2. The method of claim 1, further comprising detecting in a biological sample from the transplant recipient the expression level of at least one additional biomarker selected from the group consisting of the biomarkers disclosed in Table X and Table Z; and comparing the expression level of the at least one biomarker with the expression level of the same at least one biomarker from a biomarker control.

3. The method of claim 1 or 2, wherein the expression level of IgκV4-1, IgλL1, or IgJ is detected and compared.

4. The method of any one of the forgoing claims, wherein the biomarker control is selected from the group consisting of:

    an individual non-tolerant transplant recipient;
    a group of non-tolerant transplant recipients;
    an individual healthy non-transplant recipient;
    a group of healthy non-transplant recipients;
    a sample from the transplant recipient taken at a different time.

5. The method of claim 4, wherein the biomarker control is an individual or group of non-tolerant transplant recipients and expression level of the at least one biomarker is increased at least two-fold over the control.

6. The method any one of the forgoing claims, wherein said detection step comprises using a nucleic acid microarray or a PCR-based method.

7. The method of any one of the forgoing claims, wherein expression of at least 10 biomarkers selected from the group consisting of the biomarkers disclosed in Table X and Table Z is detected and compared.

8. The method of any one of the forgoing claims, further comprising adjusting the course of immunosuppressive therapy for the transplant recipient depending on whether the transplant recipient is predicted to be tolerant of the transplant.

9. A biomarker array comprising IgκV1D-13 and at least one biomarker selected from the group consisting of the biomarkers disclosed in Table X and Table Z.

10. The biomarker array of claim 9, comprising IgκV4-1, IgλL1, or IgJ.

11. A method of determining a course of immunosuppressive therapy for a transplant recipient, said method comprising:

    (i) detecting in a biological sample from the transplant recipient the expression level of IgκV1D-13; and
    (ii) comparing the expression level of IgκV1D-13 with the expression level of a biomarker control,
    (iii) predicting whether the transplant recipient will be tolerant of the transplant based on the comparison in step (ii); and
    (iv) adjusting the course of immunosuppressive therapy for the transplant recipient depending on whether the transplant recipient is predicted to be tolerant of the transplant.

12. The method of claim 11, further comprising detecting in a biological sample from the transplant recipient the expression level of at least one additional biomarker selected from the group consisting of the biomarkers disclosed in Table X and Table Z; and comparing the expression level of the at least one biomarker with the expression level of the same at least one biomarker from a biomarker control.

13. The method of claim 11 or 12, wherein the expression level of IgκV4-1, IgλL1, or IgJ is detected and compared.

14. A method for predicting tolerance in a transplant recipient for the transplant, the method comprising, detecting in a biological sample from the transplant recipient the amount of at least one B cell population in the sample; and comparing the amount of said at least one B cell population to the amount of the same at least one B cell population in a biological sample from a B cell control, wherein the at least one B cell population is selected from: total B cells, naive B cells, memory B cells, transitional B cells, and CD86+ B cells, thereby predicting the tolerance of the transplant recipient for the transplant.

15. The method of claim 14, wherein the B cell control is selected from the group consisting of: an individual non-tolerant transplant recipient; a group of non-tolerant transplant recipients; an individual healthy non-transplant recipient; a group of healthy non-transplant recipients; a sample from the transplant recipient taken at a different time.

# FIGURE 1

# FIGURE 2

# FIGURE 3

# FIGURE 4

Log$_2$ normalized number of molecules

# FIGURE 5

# FIGURE 6

**FIGURE 7**

FIGURE 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61182545 B
- US 6040138 A
- WO 9617958 A
- US 5807522 A
- US 5143854 A
- WO 9015070 A
- WO 9210092 A
- US 5800690 A

### Non-patent literature cited in the description

- **NANKIVELL et al.** *N Engl J Med,* 2003, vol. 349 (24), 2326-2333
- **OPELZ.** *Transplantation,* 1995, vol. 60 (11), 1220-1224
- **LECHLER et al.** *Nat Rev Immunol,* 2003, vol. 3 (2), 147-158
- **DEVLIN et al.** *Hepatology,* 1998, vol. 27 (4), 926-933
- **MAZARIEGOS et al.** *Transplantation,* 1997, vol. 63 (2), 243-249
- **KOSHIBA et al.** *Transpl Immunol,* 2007, vol. 17 (2), 94-97
- **BROUARD et al.** *Am J Transplant,* 2005, vol. 5 (2), 330-340
- **ROUSSEY-KESLER et al.** *Am J Transplant,* 2006, vol. 6 (4), 736-746
- **BROUARD et al.** *Proc Natl Acad Sci USA,* 2007, vol. 104 (39), 15448-15453
- **KINGSLEY et al.** *Transpl Int,* 2007, vol. 20 (10), 828-841
- **BROUARD et al.** Seyfert-Margolis V, Turka LA. Marking a path to transplant tolerance. *J Clin Invest,* 2007, vol. 118 (8), 2684-2686
- **MAZARIEGOS et al.** *Am J Transplant,* 2003, vol. 3 (6), 689-696
- **MARTINEZ-LLORDELLA et al.** *Am J Transplant,* 2007, vol. 7 (2), 309-319
- **MARTINEZ-LLORDELLA et al.** *J Clin Invest,* 2008, vol. 118 (8), 2845-2857
- Harrison's Principles of Internal Medicine. 2005
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Nat'l. Acad. Sci. USA,* 1988, vol. 85, 2444
- Current Protocols in Molecular Biology. Wiley Interscience, 1987
- **ALTSCHUL et al.** *Nuc. Acids Res.,* 1977, vol. 25, 3389-3402
- **ALTSCHUL et al.** *J. Mol. Biol.,* 1990, vol. 215, 403-410
- **HENIKOFF ; HENIKOFF.** *Proc. Natl. Acad. Sci. USA,* 1989, vol. 89, 10915
- **BATZER et al.** *Nucleic Acid Res.,* 1991, vol. 19, 5081
- **OHTSUKA et al.** *J. Biol. Chem.,* 1985, vol. 260, 2605-2608
- **ROSSOLINI et al.** *Mol. Cell. Probes,* 1994, vol. 8, 91-98
- **HARDIMAN.** Microarrays Methods and Applications: Nuts & Bolts. DNA Press, 2003
- **BALDI et al.** DNA Microarrays and Gene Expression: From Experiments to Data Analysis and Modeling. Cambridge University Press, 2002
- **PASTINEN.** *Genome Res.,* 1997, vol. 7, 606-614
- **JACKSON.** *Nature Biotechnology,* 1996, vol. 14, 1685
- **CHEE.** *Science,* 1995, vol. 274, 610
- **WITTRUP.** *Cytometry,* 1994, vol. 16, 206-213
- **ORCHID.** Running on Parallel Lines. *New Scientist,* 25 October 1997
- **MCCORMICK et al.** *Anal. Chem.,* 1997, vol. 69, 2626-30
- **TURGEON.** The Lab of the Future on CD-ROM?. *Medical Laboratory Management Report.,* December 1997, 1
- **ANDERSON.** Nucleic Acid Hybridization. BIOS Scientific Publishers, 1999
- **INNIS et al.** PCR Protocols, A Guide to Methods and Applications. Academic Press, Inc, 1990
- **KRONVAL et al.** *J. Immunol.,* 1973, vol. 111, 1401-1406
- **AKERSTROM et al.** *J. Immunol.,* 1985, vol. 135, 2589-2542
- Baxter-Lowe ASHI Laboratory Manual. American Society of Histocompatibility and Immunogenetics, 2002
- **GEBEL ; BRAY.** *Transplantation,* 2000, vol. 69 (7), 1370-1374

- **PEI et al.** *Hum Immunol,* 1999, vol. 60 (12), 1293-1302
- **MUTHUKUMAR et al.** *N Engl J Med,* 2005, vol. 353 (22), 2342-2351
- **STEWARD.** Immunophenotyping. John Wiley and Sons, 2000
- **ASARE et al.** *Bioinformatics,* 2009, vol. 25 (1), 48-53
- **BENJAMINI et al.** *Journal of the Royal Statistical Society,* 1995, vol. 57 (1), 289-300
- **BURGES CJC.** A Tutorial on Support Vector Machines for Pattern Recognition. *Data Mining and Knowledge Discovery,* 1998, vol. 2 (2), 121-168
- **ZHANG et al.** *BMC Bioinformatics,* 2006, vol. 7, 197
- **GOLUB et al.** *Science,* 1999, vol. 286 (5439), 531-537
- Pattern Classification and Scene Analysis. New York. John Wiley, 1973
- **HOFFMANN et al.** *J Leukoc Biol,* 2003, vol. 74 (4), 602-610
- **MATTHIAS et al.** *Nat Rev Immunol,* 2005, vol. 5 (6), 497-508
- **CUI et al.** *Neuroscience,* 2007, vol. 146 (3), 986-999
- **EVANS et al.** *J Immunol.,* 2007, vol. 178 (12), 7868-78
- **FILLATREAU et al.** *Nat Rev Immunol.,* 2008, vol. 8 (5), 391-397